# EUROPEAN PATENT APPLICATION

(11) **EP 4 194 468 A1**
(43) Date of publication of application: **14.06.2023**
(21) Application number: 21853735.5
(22) Date of filing: 06.08.2021
(51) Int. Cl.: C07K 16/28, C12N 15/13, A61K 39/395, A61P 35/00, A61P 31/04, A61P 31/10, A61P 31/12, A61P 33/02, G01N 33/574, G01N 33/68

(54) **ANTI PD-L1 ANTIBODY AND APPLICATION THEREOF**

(30) Priority: 07.08.2020 CN 202010790328
(71) Applicant: Bio-Thera Solutions, Ltd., Guangzhou, Guangdong 510530 (CN)
(72) Inventor: CHEN, Yifan, Guangzhou, Guangdong 510530 (CN); LIANG, Qiulian, Guangzhou, Guangdong 510530 (CN); YU, Jin-Chen, Guangzhou, Guangdong 510530 (CN); LI, Shengfeng, Guangzhou, Guangdong 510530 (CN)
(74) Representative: Engelhard, Markus
(86) International application number: PCT/CN2021/111320
(87) International publication number: WO 2022/028608

(57) **Abstract**

The present invention provides an anti-PD-L1 antibody and use thereof. The antibody or the antigen-binding fragment of the present invention can specifically bind to PD-L1 and block the interaction between PD-1 and PD-L1. The antibody or the antigen-binding fragment of the present invention can help the immune system to clear tumor cells, and can also be used for the diagnosis and prognosis of a tumor or a cancer.

## Description

### TECHNICAL FIELD

The present invention relates the field of bio-pharmaceuticals, and particularly to an anti-PD-L1 antibody and use thereof.

### BACKGROUND

Programmed death receptor-1 (PD-1) is a type I transmembrane glycoprotein with a molecular weight of about 55 kDa. PD-1 is an immunoinhibitory receptor expressed on activated T cells, B cells, and myeloid cells, and is a member of the CD28 immunoglobulin superfamily. As a ligand of PD-1, PD-L1 is also a type I transmembrane glycoprotein, which is widely distributed, and is expressed on the surface of antigen-presenting cells such as B cells, T cells, dendritic cells, and macrophages, as well as in tumor tissues.

The interaction between PD-1 and PD-L1 negatively regulates antigen receptor signal transduction, and weakens T cell response. So far, there are numerous studies showing that the interaction between PD-1 and PD-L1 results in a decrease in tumor-infiltrating lymphocytes, a decrease in T cell receptor-mediated proliferation, and immune evasion of cancer cells. Blocking the interaction between PD-1 and PD-L1 can increase T cell proliferation and cytokine production, improve the immunity of tumor-specific CD8+ T cells, and help the immune system to clear tumor cells.

The PD-1/PD-L1 pathway is a target for the antibody therapies in cancer treatment.

### SUMMARY

The present invention provides anti-PD-L1 antibodies or antigen-binding fragments, which can specifically bind to PD-L1, block the interaction between PD-1 and PD-L1, and help the immune system to clear tumor cells.

In some embodiments, provided is an anti-PD-L1 antibody or an antigen-binding fragment, which specifically binds to PD-L1, and comprises:
(a) an HCDR1 comprising an amino acid sequence set forth in X₁₅X₁₆X₁₇X₁₈X₁₉DSWIH, wherein X₁₅ is G, S, or N, X₁₆ is F, W, L, or S, X₁₇ is T, S, A, R, or H, X₁₈ is F, L, T, or P, and X₁₉ is S, K, R, or A; and/or
(b) an HCDR2 comprising GWISPYGGSTYYADX₂₀X₂₁X₂₂X₂₃, wherein X₂₀ is S, D, H, G, P, or Y, X₂₁ is V, F, L, M, or Y, X₂₂ is K, R, G, S, V, or H, and X₂₃ is G, H, D, Q, S, or A; and/or
(c) an HCDR3 comprising RHWPGGX₂₄X₂₅X₂₆, wherein X₂₄ is F or L, X₂₅ is D or L, and X₂₆ is Y or P.

In some embodiments, the antibody or the antigen-binding fragment specifically binds to PD-L1, and comprises:
(a) an HCDR1 comprising an amino acid sequence set forth in X₁₅X₁₆X₁₇X₁₈X₁₉DSWIH, wherein X₁₅ is G, S, or N, X₁₆ is F, W, L, or S, X₁₇ is T, S, A, R, or H, X₁₈ is F, L, T, or P, and X₁₉ is S, K, R, or A;
(b) an HCDR2 comprising GWISPYGGSTYYADX₂₀X₂₁X₂₂X₂₃, wherein X₂₀ is S, D, H, G, P, or Y, X₂₁ is V, F, L, M, or Y, X₂₂ is K, R, G, S, V, or H, and X₂₃ is G, H, D, Q, S, or A; and
(c) an HCDR3 comprising RHWPGGX₂₄X₂₅X₂₆, wherein X₂₄ is F or L, X₂₅ is D or L, and X₂₆ is Y or P.

In some embodiments, the HCDR1 comprises an amino acid sequence set forth in any one of SEQ ID NOs: 1-6 or having a variant with a substitution, deletion or insertion at a single site. In some embodiments, the HCDR2 comprises an amino acid sequence set forth in any one of SEQ ID NOs: 7-16 or having a variant with a substitution, deletion or insertion at a single site. In some embodiments, the HCDR3 comprises an amino acid sequence set forth in SEQ ID NO: 17 or 18 or having a variant with a substitution, deletion or insertion at a single site. In some embodiments, the variant with the substitution is a variant with a conservative amino acid substitution.

In some embodiments, the HCDR1 comprises an amino acid sequence set forth in SEQ ID NO: 1, the HCDR2 comprises an amino acid sequence set forth in SEQ ID NO: 16, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 7, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 8, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 9, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 10, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 11, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 12, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 13, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 14, and the HCDR3 comprises an amino acid sequence set forth in SEQ ID NO: 17.

In some embodiments, the HCDR1 comprises an amino acid sequence set forth in SEQ ID NO: 1, the HCDR2 comprises an amino acid sequence set forth in SEQ ID NO: 15, and the HCDR3 comprises an amino acid sequence set forth in SEQ ID NO: 17.

In some embodiments, the HCDR1 comprises an amino acid sequence set forth in SEQ ID NO: 2, the HCDR2 comprises an amino acid sequence set forth in SEQ ID NO: 16, and the HCDR3 comprises an amino acid sequence set forth in SEQ ID NO: 17.

In some embodiments, the HCDR1 comprises an amino acid sequence set forth in SEQ ID NO: 3, the HCDR2 comprises an amino acid sequence set forth in SEQ ID NO: 16, and the HCDR3 comprises an amino acid sequence set forth in SEQ ID NO: 17.

In some embodiments, the HCDR1 comprises an amino acid sequence set forth in SEQ ID NO: 4, the HCDR2 comprises an amino acid sequence set forth in SEQ ID NO: 16, and the HCDR3 comprises an amino acid sequence set forth in SEQ ID NO: 17.

In some embodiments, the HCDR1 comprises an amino acid sequence set forth in SEQ ID NO: 5, the HCDR2 comprises an amino acid sequence set forth in SEQ ID NO: 16, and the HCDR3 comprises an amino acid sequence set forth in SEQ ID NO: 17.

In some embodiments, the HCDR1 comprises an amino acid sequence set forth in SEQ ID NO: 6, the HCDR2 comprises an amino acid sequence set forth in SEQ ID NO: 16, and the HCDR3 comprises an amino acid sequence set forth in SEQ ID NO: 18.

In some embodiments, provided is an antibody or an antigen-binding fragment, which specifically binds to PD-L1, and comprises:
(a) an HCDR1 comprising an amino acid sequence set forth in X₁₅X₁₆X₁₇X₁₈X₁₉DSWIH, wherein X₁₅ is G, S, or N, X₁₆ is F, W, L, or S, X₁₇ is T, S, A, R, or H, X₁₈ is F, L, T, or P, and X₁₉ is S, K, R, or A; and/or
(b) an HCDR2 comprising GWISPYGGSTYYADX₂₀X₂₁X₂₂X₂₃, wherein X₂₀ is S, D, H, G, P, or Y, X₂₁ is V, F, L, M, or Y, X₂₂ is K, R, G, S, V, or H, and X₂₃ is G, H, D, Q, S, or A; and/or
(c) an HCDR3 comprising RHWPGGX₂₄X₂₅X₂₆, wherein X₂₄ is F or L, X₂₅ is D or L, and X₂₆ is Y or P; and/or
(d) an LCDR1 comprising X₁ASQX₂IX₃X₄X₅LX₆, wherein X₁ is L, Q, or R, X₂ is D, T, or G, X₃ is G or S, X₄ is K, T, or S, X₅ is H, W, F, or Y, and X₆ is N or A; and/or
(e) an LCDR2 comprising X₇ASX₈LX₉X₁₀, wherein X₇ is A or G, X₈ is T, N, S, or R, X₉ is Q or K, and X₁₀ is S or T; and/or
(f) an LCDR3 comprising QQX₁₁X₁₂X₁₃TPX₁₄T, wherein X₁₁ is Y or S, X₁₂ is Y or F, X₁₃ is S or T, and X₁₄ is R or Y

In some embodiments, the antibody or the antigen-binding fragment specifically binds to PD-L1, and comprises:
(a) an HCDR1 comprising an amino acid sequence set forth in X₁₅X₁₆X₁₇X₁₈X₁₉DSWIH, wherein X₁₅ is G, S, or N, X₁₆ is F, W, L, or S, X₁₇ is T, S, A, R, or H, X₁₈ is F, L, T, or P, and X₁₉ is S, K, R, or A;
(b) an HCDR2 comprising GWISPYGGSTYYADX₂₀X₂₁X₂₂X₂₃, wherein X₂₀ is S, D, H, G, P, or Y, X₂₁ is V, F, L, M, or Y, X₂₂ is K, R, G, S, V, or H, and X₂₃ is G, H, D, Q, S, or A;
(c) an HCDR3 comprising RHWPGGX₂₄X₂₅X₂₆, wherein X₂₄ is F or L, X₂₅ is D or L, and X₂₆ is Y or P;
(d) an LCDR1 comprising X₁ASQX₂IX₃X₄X₅LX₆, wherein X₁ is L, Q, or R, X₂ is D, T, or G, X₃ is G or S, X₄ is K, T, or S, X₅ is H, W, F, or Y, and X₆ is N or A;
(e) an LCDR2 comprising X₇ASX₈LX₉X₁₀, wherein X₇ is A or G, X₈ is T, N, S, or R, X₉ is Q or K, and X₁₀ is S or T; and
(f) an LCDR3 comprising QQX₁₁X₁₂X₁₃TPX₁₄T, wherein X₁₁ is Y or S, X₁₂ is Y or F, X₁₃ is S or T, and X₁₄ is R or Y

In some embodiments, the LCDR1 comprises an amino acid sequence set forth in any one of SEQ ID NOs: 19-23 or having a variant with a substitution, deletion or insertion at a single site. In some embodiments, the LCDR2 comprises an amino acid sequence set forth in any one of SEQ ID NOs: 24-27 or having a variant with a substitution, deletion or insertion at a single site. In some embodiments, the LCDR3 comprises an amino acid sequence set forth in any one of SEQ ID NOs: 28-31 or having a variant with a substitution, deletion or insertion at a single site. In some embodiments, the variant with the substitution is a variant with a conservative amino acid substitution.

In some embodiments, the LCDR1 comprises an amino acid sequence set forth in SEQ ID NO: 19, the LCDR2 comprises an amino acid sequence set forth in SEQ ID NO: 24, and the LCDR3 comprises an amino acid sequence set forth in SEQ ID NO: 28.

In some embodiments, the LCDR1 comprises an amino acid sequence set forth in SEQ ID NO: 20, the LCDR2 comprises an amino acid sequence set forth in SEQ ID NO: 25, and the LCDR3 comprises an amino acid sequence set forth in SEQ ID NO: 29.

In some embodiments, the LCDR1 comprises an amino acid sequence set forth in SEQ ID NO: 21, the LCDR2 comprises an amino acid sequence set forth in SEQ ID NO: 26, and the LCDR3 comprises an amino acid sequence set forth in SEQ ID NO: 30.

In some embodiments, the LCDR1 comprises an amino acid sequence set forth in SEQ ID NO: 22, the LCDR2 comprises an amino acid sequence set forth in SEQ ID NO: 27, and the LCDR3 comprises an amino acid sequence set forth in SEQ ID NO: 31.

In some embodiments, the LCDR1 comprises an amino acid sequence set forth in SEQ ID NO: 23, the LCDR2 comprises an amino acid sequence set forth in SEQ ID NO: 26, and the LCDR3 comprises an amino acid sequence set forth in SEQ ID NO: 30.

In some embodiments, the LCDR1 comprises an amino acid sequence set forth in SEQ ID NO: 19, the LCDR2 comprises an amino acid sequence set forth in SEQ ID NO: 26, and the LCDR3 comprises an amino acid sequence set forth in SEQ ID NO: 31.

In some embodiments, provided is an antibody or an antigen-binding fragment, which specifically binds to PD-L1, and comprises: (a) an HCDR1 comprising an amino acid sequence set forth in any one of SEQ ID NOs: 1-6 or having a variant with a substitution, deletion or insertion at a single site; and/or (b) an HCDR2 comprising an amino acid sequence set forth in any one of SEQ ID NOs: 7-16 or having a variant with a substitution, deletion or insertion at a single site; and/or (c) an HCDR3 comprising an amino acid sequence set forth in SEQ ID NO: 17 or 18 or having a variant with a substitution, deletion or insertion at a single site; and/or (d) an LCDR1 comprising an amino acid sequence set forth in any one of SEQ ID NOs: 19-23 or having a variant with a substitution, deletion or insertion at a single site; and/or (e) an LCDR2 comprising an amino acid sequence set forth in any one of SEQ ID NOs: 24-27 or having a variant with a substitution, deletion or insertion at a single site; and/or (f) an LCDR3 comprising an amino acid sequence set forth in any one of SEQ ID NOs: 28-31 or having a variant with a substitution, deletion or insertion at a single site.

In some embodiments, the antibody or the antigen-binding fragment specifically binds to PD-L1, and the antibody or the antigen-binding fragment at least comprises one, two, three, four, five, or all of the HCDR1 set forth in SEQ ID NO: 1, the HCDR2 set forth in SEQ ID NO: 16, the HCDR3 set forth in SEQ ID NO: 17, the LCDR1 set forth in any one of SEQ ID NOs: 19-23, the LCDR2 set forth in any one of SEQ ID NOs: 24-27, and the LCDR3 set forth in any one of SEQ ID NOs: 28-31.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 20, the LCDR2 set forth in SEQ ID NO: 25, and the LCDR3 set forth in SEQ ID NO: 29.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 21, the LCDR2 set forth in SEQ ID NO: 26, and the LCDR3 set forth in SEQ ID NO: 30.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 22, the LCDR2 set forth in SEQ ID NO: 27, and the LCDR3 set forth in SEQ ID NO: 31.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 23, the LCDR2 set forth in SEQ ID NO: 26, and the LCDR3 set forth in SEQ ID NO: 30.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 19, the LCDR2 set forth in SEQ ID NO: 26, and the LCDR3 set forth in SEQ ID NO: 31.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 7, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 8, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 9, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 10, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28.

In some embodiments, the antibody or the antigen-binding fragment specifically binds to PD-L1, and the antibody or the antigen-binding fragment at least comprises the HCDR1 set forth in SEQ ID NO: 1, the HCDR2 set forth in SEQ ID NO: 11, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28.

In some embodiments, a heavy chain of the antibody or the antigen-binding fragment further comprises FR1, FR2, FR3, and FR4, wherein the heavy chain FR1 comprises a sequence set forth in SEQ ID NO: 32, a sequence having at least 90% identity to the sequence set forth in SEQ ID NO: 32, or an amino acid sequence having one or more conservative amino acid substitutions as compared to the sequence set forth in SEQ ID NO: 32; and/or
the heavy chain FR2 of the antibody or the antigen-binding fragment comprises a sequence set forth in SEQ ID NO: 33, a sequence having at least 90% identity to the sequence set forth in SEQ ID NO: 33, or an amino acid sequence having one or more conservative amino acid substitutions as compared to the sequence set forth in SEQ ID NO: 33; and/or
the heavy chain FR3 of the antibody or the antigen-binding fragment comprises a sequence set forth in SEQ ID NO: 34, a sequence having at least 90% identity to the sequence set forth in SEQ ID NO: 34, or an amino acid sequence having one or more conservative amino acid substitutions as compared to the sequence set forth in SEQ ID NO: 34; and/or
the heavy chain FR4 of the antibody or the antigen-binding fragment comprises a sequence set forth in SEQ ID NO: 35, a sequence having at least 90% identity to the sequence set forth in SEQ ID NO: 35, or an amino acid sequence having one or more conservative amino acid substitutions as compared to the sequence set forth in SEQ ID NO: 35.

In some embodiments, the heavy chain FR1 of the antibody or the antigen-binding fragment comprises a sequence set forth in SEQ ID NO: 32, the heavy chain FR2 of the antibody or the antigen-binding fragment comprises a sequence set forth in SEQ ID NO: 33, the heavy chain FR3 of the antibody or the antigen-binding fragment comprises a sequence set forth in SEQ ID NO: 34, and the heavy chain FR4 of the antibody or the antigen-binding fragment comprises a sequence set forth in SEQ ID NO: 35.

In some embodiments, a heavy chain variable region (VH) comprises the structure FR1-HCDR1-FR2-HCDR2-FR3-HCDR3-FR4. In some embodiments, the heavy chain variable region of the antibody or the antigen-binding fragment comprises a sequence set forth in any one of SEQ ID NOs: 36-50, a sequence having at least 80% identity to the sequence set forth in any one of SEQ ID NOs: 36-50, or an amino acid sequence having one or more conservative amino acid substitutions as compared to the sequence set forth in any one of SEQ ID NOs: 36-50.

In some embodiments, a light chain of the antibody or the antigen-binding fragment further comprises FR1, FR2, FR3, and FR4, wherein the light chain FR1 comprises a sequence set forth in any one of SEQ ID NOs: 51-56, a sequence having at least 90% identity to the sequence set forth in any one of SEQ ID NOs: 51-56, or an amino acid sequence having one or more conservative amino acid substitutions as compared to the sequence set forth in any one of SEQ ID NOs: 51-56; and/or
the light chain FR2 of the antibody or the antigen-binding fragment comprises a sequence set forth in any one of SEQ ID NOs: 57-60, a sequence having at least 90% identity to the sequence set forth in any one of SEQ ID NOs: 57-60, or an amino acid sequence having one or more conservative amino acid substitutions as compared to the sequence set forth in any one of SEQ ID NOs: 57-60; and/or
the light chain FR3 of the antibody or the antigen-binding fragment comprises a sequence set forth in any one of SEQ ID NOs: 61-64, a sequence having at least 90% identity to the sequence set forth in any one of SEQ ID NOs: 61-64, or an amino acid sequence having one or more conservative amino acid substitutions as compared to the sequence set forth in any one of SEQ ID NOs: 61-64; and/or
the light chain FR4 of the antibody or the antigen-binding fragment comprises a sequence set forth in any one of SEQ ID NOs: 65-67, a sequence having at least 90% identity to the sequence set forth in any one of SEQ ID NOs: 65-67, or an amino acid sequence having one or more conservative amino acid substitutions as compared to the sequence set forth in any one of SEQ ID NOs: 65-67.

In some embodiments, the light chain FR1 of the antibody or the antigen-binding fragment comprises a sequence set forth in SEQ ID NO: 51, the light chain FR2 of the antibody or the antigen-binding fragment comprises a sequence set forth in SEQ ID NO: 57, the light chain FR3 of the antibody or the antigen-binding fragment comprises a sequence set forth in SEQ ID NO: 61, and the light chain FR4 of the antibody or the antigen-binding fragment comprises a sequence set forth in SEQ ID NO: 65.

In some embodiments, the light chain FR1 of the antibody or the antigen-binding fragment comprises a sequence set forth in SEQ ID NO: 52, the light chain FR2 of the antibody or the antigen-binding fragment comprises a sequence set forth in SEQ ID NO: 58, the light chain FR3 of the antibody or the antigen-binding fragment comprises a sequence set forth in SEQ ID NO: 62, and the light chain FR4 of the antibody or the antigen-binding fragment comprises a sequence set forth in SEQ ID NO: 66.

In some embodiments, the light chain FR1 of the antibody or the antigen-binding fragment comprises a sequence set forth in SEQ ID NO: 53, the light chain FR2 of the antibody or the antigen-binding fragment comprises a sequence set forth in SEQ ID NO: 59, the light chain FR3 of the antibody or the antigen-binding fragment comprises a sequence set forth in SEQ ID NO: 63, and the light chain FR4 of the antibody or the antigen-binding fragment comprises a sequence set forth in SEQ ID NO: 67.

In some embodiments, the light chain FR1 of the antibody or the antigen-binding fragment comprises a sequence set forth in SEQ ID NO: 54, the light chain FR2 of the antibody or the antigen-binding fragment comprises a sequence set forth in SEQ ID NO: 60, the light chain FR3 of the antibody or the antigen-binding fragment comprises a sequence set forth in SEQ ID NO: 64, and the light chain FR4 of the antibody or the antigen-binding fragment comprises a sequence set forth in SEQ ID NO: 67.

In some embodiments, the light chain FR1 of the antibody or the antigen-binding fragment comprises a sequence set forth in SEQ ID NO: 55, the light chain FR2 of the antibody or the antigen-binding fragment comprises a sequence set forth in SEQ ID NO: 59, the light chain FR3 of the antibody or the antigen-binding fragment comprises a sequence set forth in SEQ ID NO: 63, and the light chain FR4 of the antibody or the antigen-binding fragment comprises a sequence set forth in SEQ ID NO: 67.

In some embodiments, the light chain FR1 of the antibody or the antigen-binding fragment comprises a sequence set forth in SEQ ID NO: 56, the light chain FR2 of the antibody or the antigen-binding fragment comprises a sequence set forth in SEQ ID NO: 57, the light chain FR3 of the antibody or the antigen-binding fragment comprises a sequence set forth in SEQ ID NO: 61, and the light chain FR4 of the antibody or the antigen-binding fragment comprises a sequence set forth in SEQ ID NO: 67.

In some embodiments, a light chain variable region (VL) comprises the structure FR1-LCDR1-FR2-LCDR2-FR3-LCDR3-FR4. In some embodiments, the light chain variable region of the antibody or the antigen-binding fragment comprises a sequence set forth in any one of SEQ ID NOs: 68-73, a sequence having at least 80% identity to the sequence set forth in any one of SEQ ID NOs: 68-73, or an amino acid sequence having one or more conservative amino acid substitutions as compared to the sequence set forth in any one of SEQ ID NOs: 68-73.

In some embodiments, the heavy chain variable region of the antibody or the antigen-binding fragment comprises a sequence set forth in any one of SEQ ID NOs: 36-50, and the light chain variable region of the antibody or the antigen-binding fragment comprises a sequence set forth in any one of SEQ ID NOs: 68-73.

In some embodiments, the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 68.

In some embodiments, the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 69.

In some embodiments, the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 70.

In some embodiments, the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 71.

In some embodiments, the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 72.

In some embodiments, the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 73.

In some embodiments, the heavy chain variable region of the antibody or the antigen-binding fragment comprises a sequence set forth in SEQ ID NO: 37, and the light chain variable region of the antibody or the antigen-binding fragment comprises a sequence set forth in SEQ ID NO: 68.

In some embodiments, the heavy chain variable region of the antibody or the antigen-binding fragment comprises a sequence set forth in SEQ ID NO: 38, and the light chain variable region of the antibody or the antigen-binding fragment comprises a sequence set forth in SEQ ID NO: 68.

In some embodiments, the heavy chain variable region of the antibody or the antigen-binding fragment comprises a sequence set forth in SEQ ID NO: 39, and the light chain variable region of the antibody or the antigen-binding fragment comprises a sequence set forth in SEQ ID NO: 68.

In some embodiments, the heavy chain variable region of the antibody or the antigen-binding fragment comprises a sequence set forth in SEQ ID NO: 40, and the light chain variable region of the antibody or the antigen-binding fragment comprises a sequence set forth in SEQ ID NO: 68.

In some embodiments, the heavy chain variable region of the antibody or the antigen-binding fragment comprises a sequence set forth in SEQ ID NO: 41, and the light chain variable region of the antibody or the antigen-binding fragment comprises a sequence set forth in SEQ ID NO: 68.

In some embodiments, the antibody or the antigen-binding fragment further comprises a heavy chain constant region, a light chain constant region, an Fc region, or a combination thereof. In some embodiments, the light chain constant region is a κ or λ chain constant region. In some embodiments, the antibody or the fragment thereof is of one of the isotypes IgG, IgM, IgA, IgE or IgD. In some embodiments, the isotype is IgG1, IgG2, IgG3 or IgG4. In some embodiments, the antibody or the antigen-binding fragment is a murine antibody, a chimeric antibody, a humanized antibody, or a fully humanized antibody.

In some embodiments, the Fc is a variant Fc region. In some embodiments, the variant Fc region has one or more amino acid modifications, such as substitutions, deletions, or insertions, relative to a parent Fc region. In some embodiments, the amino acid modification of the Fc region alters effector function activity, relative to the activity of the parent Fc region. In some embodiments, the variant Fc region can have altered (i.e., increased or decreased) antibody-dependent cellular cytotoxicity (ADCC), complement-dependent cytotoxicity (CDC), phagocytosis, opsonization, or cell binding. In some embodiments, the amino acid modification of the Fc region can alter the affinity of the variant Fc region for an FcyR (Fcy receptor), relative to the parent Fc region. In some embodiments, the Fc region is derived from IgG1 or IgG4. In some embodiments, a mutation of the Fc region is N297A.

In some embodiments, the antibody or the antigen-binding fragment is an isolated antibody or an antigen-binding fragment. In some embodiments, the antibody or the antigen-binding fragment is an scFV, an Fab, an F(ab)₂, or an IgG1. In some embodiments, the antibody or the antigen-binding fragment is a monoclonal antibody.

In some embodiments, the heavy chain constant region of the antibody or the antigen-binding fragment comprises a sequence having an amino acid sequence set forth in SEQ ID NO: 74 or 75, a sequence having at least 80% identity to the sequence set forth in SEQ ID NO: 74 or 75, or an amino acid sequence having one or more conservative amino acid substitutions as compared to the sequence set forth in SEQ ID NO: 74 or 75; and/or
the light chain constant region of the antibody or the antigen-binding fragment comprises a sequence having an amino acid sequence set forth in SEQ ID NO: 76, a sequence having at least 80% identity to the sequence set forth in SEQ ID NO: 76, or an amino acid sequence having one or more conservative amino acid substitutions as compared to the sequence set forth in SEQ ID NO: 76.

In some embodiments, the heavy chain constant region of the antibody or the antigen-binding fragment comprises a sequence having an amino acid sequence set forth in SEQ ID NO: 74, and the light chain constant region of the antibody or the antigen-binding fragment comprises a sequence having an amino acid sequence set forth in SEQ ID NO: 76. In some embodiments, the heavy chain constant region of the antibody or the antigen-binding fragment comprises a sequence having an amino acid sequence set forth in SEQ ID NO: 75, and the light chain constant region of the antibody or the antigen-binding fragment comprises a sequence having an amino acid sequence set forth in SEQ ID NO: 76.

In some embodiments, a heavy chain of the antibody comprises a sequence having an amino acid sequence set forth in any one of SEQ ID NOs: 77-91, a sequence having at least 80% identity to the sequence set forth in any one of SEQ ID NOs: 77-91, or an amino acid sequence having one or more conservative amino acid substitutions as compared to the sequence set forth in any one of SEQ ID NOs: 77-91; and/or
a light chain of the antibody comprises a sequence having an amino acid sequence set forth in any one of SEQ ID NOs: 92-97, a sequence having at least 80% identity to the sequence set forth in any one of SEQ ID NOs: 92-97, or an amino acid sequence having one or more conservative amino acid substitutions as compared to the sequence set forth in SEQ ID NOs: 92-97.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 92.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 93.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 94.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 95.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 96.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 97.

In some embodiments, the heavy chain of the antibody comprises a sequence having an amino acid sequence set forth in SEQ ID NO: 78, and the light chain of the antibody comprises a sequence having an amino acid sequence set forth in SEQ ID NO: 92.

In some embodiments, the heavy chain of the antibody comprises a sequence having an amino acid sequence set forth in SEQ ID NO: 79, and the light chain of the antibody comprises a sequence having an amino acid sequence set forth in SEQ ID NO: 92.

In some embodiments, the heavy chain of the antibody comprises a sequence having an amino acid sequence set forth in SEQ ID NO: 80, and the light chain of the antibody comprises a sequence having an amino acid sequence set forth in SEQ ID NO: 92.

In some embodiments, the heavy chain of the antibody comprises a sequence having an amino acid sequence set forth in SEQ ID NO: 81, and the light chain of the antibody comprises a sequence having an amino acid sequence set forth in SEQ ID NO: 92.

In some embodiments, the heavy chain of the antibody comprises a sequence having an amino acid sequence set forth in SEQ ID NO: 82, and the light chain of the antibody comprises a sequence having an amino acid sequence set forth in SEQ ID NO: 92.

In one embodiment, the antibody or the antigen-binding fragment is a monoclonal antibody (including a full-length monoclonal antibody), a polyclonal antibody, or a multispecific antibody or an antigen-binding fragment (e.g., a bispecific antibody or an antigen-binding fragment).

In some embodiments, the antibody has two identical heavy chains and two identical light chains, and the Fc regions pair to form disulfide bonds.

In one embodiment, the antibody fragment (antigen-binding fragment) is an Fab antibody, an Fv antibody, or an scFv antibody.

The present invention further provides a nucleic acid molecule encoding the antibody or the antigen-binding fragment. In some embodiments, the nucleic acid molecule is an isolated nucleic acid molecule.

The present invention further provides a vector comprising the nucleic acid molecule. In some embodiments, the vector is an isolated vector. In some embodiments, the vector comprising the nucleic acid molecule is a nucleic acid fragment, a plasmid, a phage, or a virus.

The present invention further provides a host cell comprising the nucleic acid molecule or the vector. In some embodiments, the host cell is an isolated host cell. In some embodiments, the host cell is a CHO cell, a HEK cell (e.g., a HEK293F cell), a BHK cell, a Cos1 cell, a Cos7 cell, a CV1 cell, or a murine L cell.

The present invention further provides a pharmaceutical composition comprising the antibody or the antigen-binding fragment described above, and a pharmaceutically acceptable excipient.

The present invention further provides a treatment method and use. In some embodiments, provided is a method for treating or ameliorating a T cell dysfunctional disorder, wherein the method comprises administering to a patient an effective dose of the antibody or the antigen-binding fragment. In some embodiments, provided is use of the antibody or the antigen-binding fragment in the treatment or amelioration of a T cell dysfunctional disorder. In some embodiments, provided is use of the antibody or the antigen-binding fragment in the manufacture of a medicament for treating or ameliorating T cell dysfunction.

In some embodiments, the T cell dysfunctional disorder includes, but is not limited to, an infection caused by bacteria, viruses, fungi or protozoa, as well as a cancer and a tumor. In some embodiments, the cancer and the tumor include, but are not limited to, breast cancer, lung cancer, colon cancer, ovarian cancer, melanoma, bladder cancer, renal cancer, liver cancer, salivary gland cancer, gastric cancer, neuroglioma, thyroid cancer, thymus cancer, epithelial cancer, head cancer, neck cancer, and pancreatic cancer.

The present invention further provides a diagnostic method and use. In some embodiments, provided is a method for detecting PD-L1 expression in a sample, wherein the method comprises contacting the sample with the antibody or the antigen-binding fragment such that the antibody or the antigen-binding fragment binds to PD-L1, and detecting the binding, i.e., an amount of PD-L1 in the sample. In some embodiments, provided is use of the antibody or the antigen-binding fragment in the manufacture of a kit for the diagnosis or prognosis of a cancer or a tumor. In some embodiments, provided is a diagnostic or prognostic kit comprising the antibody or the antigen-binding fragment.

The present invention provides an anti-PD-L1 antibody or an antigen-binding fragment and use thereof. The antibody or the antigen-binding fragment of the present invention can specifically bind to PD-L1, block the interaction between PD-1 and PD-L1, and help the immune system to clear tumor cells. The antibody or the antigen-binding fragment of the present invention can be used for treating or ameliorating a T cell dysfunctional disorder, and can also be used for the diagnosis and prognosis of a cancer or a tumor.

### BRIEF DESCRIPTION OF THE DRAWINGS

FIG. 1 shows that anti-PD-L1 antibodies block the PD-1/PD-L1 interaction; in FIGs. 1A and 1B, the ordinate represents fluorescence value (relative light unit), and the abscissa represents concentration.
FIG. 2 shows the binding of anti-PD-L1 antibodies to cells overexpressing PD-L1; wherein the ordinate represents mean fluorescence intensity, the abscissa represents concentration, and Isotype represents a negative control antibody IgG-Isotype.
FIG. 3A shows the effect of the antibody on tumor volume.
FIG. 3B shows the effect of the antibody on tumor weight.
FIG. 3C shows the effect of the antibody on mouse body weight.
FIG. 4 shows the binding of the scFv against PD-L1 to PD-L1-His-Biotin detected by flow cytometry.

### Terms

Unless otherwise specified, each of the following terms shall have the meaning described below.

### Definitions

It should be noted that the term "an" entity refers to one or more of the entities. For example, "an antibody" should be interpreted as one or more antibodies. As such, the terms "a" (or "an"), "one or more" and "at least one" can be used interchangeably herein.

As used herein, the term "contain" or "comprise" means that the compositions, methods and the like comprise the recited elements, such as components or steps and do not exclude the others. "Consisting essentially of..." means that the compositions and methods exclude other elements that have a fundamental impact on the characteristics of the combination, but do not exclude elements that do not substantially affect the characteristics of the compositions or methods. "Consisting of" means excluding elements not specifically listed.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a particular length of the product. Thus, included within the definition of "polypeptide" are peptides, dipeptides, tripeptides, oligopeptides, "proteins", "amino acid chains", or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage or non-naturally occurring amino acid modification. The polypeptide may be derived from a natural biological source or produced by recombinant techniques, but it is not necessarily translated from a specified nucleic acid sequence, and it may be produced in any manner, including chemical synthesis.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The same amino acid is encoded by different codons, which is known as "codon degeneracy". Amino acids include natural amino acids and non-natural amino acids. Natural amino acids include alanine (three-letter code: ala, one-letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

"Conservative amino acid substitution" refers to the substitution of one amino acid residue with another amino acid residue having a side chain (R group) of similar chemical nature (e.g., charge or hydrophobicity). Generally, conservative amino acid substitutions do not substantially alter the functional properties of the protein. Examples of amino acid classes with a side chain of similar chemical nature include: 1) an aliphatic side chain: glycine, alanine, valine, leucine and isoleucine; 2) an aliphatic hydroxyl side chain: serine and threonine; 3) an amide-containing side chain: asparagine and glutamine; 4) an aromatic side chain: phenylalanine, tyrosine and tryptophan; 5) a basic side chain: lysine, arginine and histidine; and 6) an acidic side chain: aspartic acid and glutamic acid.

A number of amino acids of "conservative amino acid substitutions of FR1, FR2, FR3 and FR4 in the framework regions" is about 1, about 2, about 3, about 4, or about 5 conservative amino acid substitutions. A number of amino acids of "conservative amino acid substitutions of VL or VH" is about 1, about 2, about 3, about 4, about 5, about 6, about 8, about 9, about 10, about 11, about 13, about 14, about 15 conservative amino acid substitutions, or a range between any two of these values (inclusive) or any value therein. A number of amino acids of "conservative amino acid substitutions of a heavy chain constant region, a light chain constant region, a heavy light or a light chain" is about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

The term "isolated" as used herein with respect to cells, nucleic acids, polypeptides, antibodies and the like, e.g., "isolated" DNA, RNA, polypeptides, or antibodies, refers to molecules that are separated from one or more other components, e.g., DNA or RNA, in the natural environment of the cell. The term "isolated" as used herein also refers to nucleic acids or peptides that are substantially free of cellular materials, viral materials or cell culture media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides, antibodies and the like are usually prepared by at least one purification step. In some embodiments, the purity of the isolated nucleic acids, polypeptides, antibodies and the like is at least about 50%, about 60%, about 70%, about 80%, about 90%, about 95%, about 99%, or a range between any two of these values (inclusive) or any value therein.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Homology", "identity" or "similarity" refers to sequence similarity between two peptides or between two nucleic acid molecules. Homology can be determined by comparing the positions that can be aligned in the sequences. When a position of the compared sequences is occupied by the same base or amino acid, then the molecules are homologous at that position. The degree of homology between the sequences is a function of the number of matching or homologous positions shared by the sequences.

In some embodiments, "at least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein.

In some embodiments, "at least 90% identity" refers to about 90% identity, about 91% identity, about 92% identity, about 93% identity, about 95% identity, about 96% identity, about 97% identity, about 98% identity, about 99% identity, or a range between any two of these values (inclusive) or any value therein.

A polynucleotide or a polynucleotide sequence (or a polypeptide or an antibody sequence) having a certain percentage (e.g., 90%, 95%, 98% or 99%) of "identity or sequence identity" to another sequence refers to that when the sequences are aligned, the percentage of bases (or amino acids) in the sequence are the same. This alignment and identity percentage or sequence identity can be determined using visual inspection or software programs known in the art, such as the software programs described in Ausubel et al. eds., (2007), Current Protocols in Molecular Biology*.* Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above-specified percentage identity and encoding polypeptides having identical or similar biological activity.

A polynucleotide consists of a specific sequence of four nucleotide bases: adenine (A), cytosine (C), guanine (G), thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transport RNA, ribosomal RNA, ribozymes, cDNA, dsRNA, siRNA, miRNA, recombinant polynucleotides, branched polynucleotides, plasmids, vectors, isolated DNA of any sequence, isolated RNA of any sequence, nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

The term "encoding" as applied to a polynucleotide refers to a polynucleotide known to "encode" a polypeptide. When in its native state or manipulated by methods well known to those skilled in the art, the polypeptide and a fragment thereof can be produced by transcript and/or translation.

"Antibody" or "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising at least a portion of an immunoglobulin molecule that has biological activity for binding to an antigen. The antibody and the antigen-binding fragment include, but are not limited to, complementarity determining regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein. The CDRs include light chain CDRs (LCDR1-3) and heavy chain CDRs (HCDR1-3). An antibody or an antigen-binding fragment can specifically recognize and bind to one or more (e.g., two) polypeptides or polypeptide complexes of antigens. The antibody or the antigen-binding fragment that specifically recognizes and binds to multiple (e.g., two) antigens can be referred to as a multispecific (e.g., bispecific) antibody or an antigen-binding fragment.

The term "antibody fragment" or "antigen-binding fragment" refers to a part of an antibody, and the composition of the antibody fragment of the present invention may be similar to F(ab')₂, F(ab)₂, Fab', Fab, Fv, scFv and the like in monospecific antibody fragments. Regardless of its structure, the antibody fragment binds to the same antigen recognized by an intact antibody. The term "antibody fragment" includes aptamers, mirror isomers, and bivalent antibodies. The term "antigen-binding fragment" also includes any synthetic or genetically engineered protein that functions as an antibody by binding to a specific antigen to form a complex.

"Single chain variable fragment" or "scFv" refers to a fusion protein of a heavy chain variable region (VH) and a light chain variable region (VL) of an immunoglobulin. In some aspects, these regions are linked to a short linker peptide having 10 to about 25 amino acids. The linker may be enriched with glycine to improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

The term "antibody" includes a wide variety of polypeptides that can be biochemically distinguished. Those skilled in the art will appreciate that the classes of heavy chains include gamma, mu, alpha, delta, or epsilon (γ, µ, α, δ, or ε), and some subclasses (e.g., γ1-γ4). The nature of this chain determines the "type" of the antibody as IgG, IgM, IgA, IgD or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In some embodiments, the immunoglobulin molecule is an IgG species. These four chains are connected in a "Y" configuration through disulfide bonds, wherein the light chain starts at the opening of "Y" configuration and extends through the variable region to surround the heavy chain.

Antibodies, antigen-binding fragments or derivatives disclosed herein include, but are not limited to, polyclonal, monoclonal, multispecific, fully human, humanized, primatized or chimeric antibodies, single-chain antibodies, epitope-binding fragments (e.g., Fab, Fab' and F(ab')₂), and scFv.

Light chains can be classified into kappa (κ) or lambda (λ). Each heavy chain may bind to a κ or λ light chain. In general, when an immunoglobulin is produced by a hybridoma, a B cell or a genetically engineered host cell, the light and heavy chains are bound by covalent bonds, and the "tail" portions of the two heavy chains are bound by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is Vκ; the immunoglobulin λ light chain variable region is V_{λ}.

The light chain variable region (VL) and heavy chain variable region (VH) determine the antigen recognition and specificity. The light chain constant region and the heavy chain constant region impart important biological properties such as secretion, transplacental movement, Fc receptor binding, and complement binding. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region, exhibit little intermolecular variability. Most of the framework regions adopt a β-sheet conformation, with the CDRs forming a loop structure connected to, or in some cases forming part of, the β-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. For given heavy or light chain variable region, amino acids comprising the CDRs and the framework regions may be identified by one of ordinary skills in the art according to known method (see, Kabat, E., et al., U.S. Department of Health and Human Services, Sequences of Proteins of Immunological Interest, (1983) and Chothia and Lesk, J. Mol. Biol., 196:901-917 (1987)).

Where the terms used and/or accepted in the art have two or more definitions, the definitions of the terms used herein include all of these meanings unless explicitly indicated as opposite.

One specific example is the use of the term "complementarity determining regions" ("CDRs") to describe non-contiguous antigen-binding sites found within the variable regions of heavy and light chain polypeptides. This specific region is described in Kabat et al., U.S. Dept. of Health and Human Services, Sequences of Proteins of Immunological Interest (1983) and Chothia et al., J. Mol. Biol. 196:901-917 (1987), which are incorporated herein by reference in their entireties.

CDRs defined according to Kabat and Chothia schemes include overlaps or subsets of amino acid residues when compared to each other. Nevertheless, it is within the scope of the present invention to apply any definition to refer to the CDRs of an antibody or a variant thereof. The exact number of residues comprising a specific CDR will vary according to the sequence and size of the CDR. Those skilled in the art can generally determine which specific residues a CDR comprises based on the variable region amino acid sequence of an antibody.

Kabat et al. also define a numbering system applicable to the variable region sequence of any antibody. One of ordinary skills in the art can apply the "Kabat numbering" system to any variable region sequence without depending on other experimental data beyond the sequence itself. "Kabat numbering" refers to the numbering system proposed by Kabat et al., U.S. Dept. of Health and Human Services in Sequence of Proteins of Immunological Interest (1983). EU or Chothia numbering system can be also applicable to the antibody.

The antibodies disclosed herein may be derived from any animal, including birds and mammals. Preferably, the antibody is derived from a human, a mouse, a donkey, a rabbit, a goat, a camel, a llama, a horse, or a chicken source. In another embodiment, the variable region may be derived from a condricthoid source (e.g., from a shark).

The heavy chain constant region comprises at least one of a CH1 domain, a hinge (e.g., upper, middle, and/or lower hinge region) domain, a CH2 domain, a CH3 domain, or a variant or a fragment. The heavy chain constant region of the antibody may be derived from different immunoglobulin molecules. For example, the heavy chain constant region of a polypeptide may comprise a CH1 domain derived from an IgG₁ molecule and a hinge region derived from an IgG₃ molecule. In another embodiment, the heavy chain constant region may comprise a hinge region derived partially from an IgG₁ molecule and partially from an IgG₃ molecule. In another embodiment, a portion of the heavy chain may comprise a chimeric hinge region derived partially from an IgG₁ molecule and partially from an IgG₄ molecule.

"Light chain constant region" includes a part of amino acid sequence from the light chain of an antibody. Preferably, the light chain constant region comprises at least one of a constant κ domain or a constant λ domain. A "light chain-heavy chain pair" refers to a collection of light and heavy chains that can form dimers through disulfide bonds between the CL domain of the light chain and the CH1 domain of the heavy chain.

"VH domain" includes the variable domain at the amino terminus of an immunoglobulin heavy chain, and "CH1 domain" includes the first constant region (mostly at the amino terminus) of an immunoglobulin heavy chain. The CH2 domain is not closely paired with other domains, but rather two N-linked branched carbohydrate chains are inserted between the two CH2 domains of an intact native IgG molecule. It is also documented that the CH3 domain extends from the CH2 domain to the C terminus of the IgG molecule, and comprises about 108 residues. "Hinge region" includes a portion of the heavy chain region connecting the CH1 domain and CH2 domain. The hinge region comprises about 25 residues and is flexible, thereby enabling independent movement of the two N-terminal antigen-binding regions. The hinge region can be subdivided into three distinct domains: upper, middle and lower hinge domains (Roux et al., J. Immunol., 161:4083 (1998)).

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

A "chimeric antibody" refers to any antibody in which the variable region of the antibody is obtained or derived from a first species, and constant region thereof (which may be intact, partial, or modified) is derived from a second species. In certain embodiments, the variable region is derived from a non-human source (e.g., mouse or primate) and the constant region is derived from a human source.

"Specifically bind to" or "specific for" generally refers to that an antibody or an antigen-binding fragment and a specific antigen bind to an epitope through its antigen-binding domain to form a relatively stable complex. "Specificity" can be expressed by relative affinity of an antibody or an antigen-binding fragment for binding to a specific antigen or epitope. For example, an antibody "A" can be considered to have a higher specificity for an antigen than an antibody "B" if the antibody "A" has a greater relative affinity for the antigen than the antibody "B". Specific binding can be described by the equilibrium dissociation constant (KD). A smaller KD means a tighter binding. Methods for determining whether two molecules specifically bind to each other are well known in the art, and include, for example, equilibrium dialysis, surface plasmon resonance, biofilm layer interferometry, and the like. Antibodies that "specifically bind" to antigen a include antibodies that have an equilibrium dissociation constant KD of less than or equal to about 100 nM, less than or equal to about 10 nM, less than or equal to about 5 nM, or less than or equal to about 1 nM with antigen a.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total), prolongation of life expectancy in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the pharmaceutical composition disclosed herein for detection, diagnostic procedures, and/or treatment.

"Patient" refers to any mammal in need of diagnosis, prognosis or treatment, including humans, dogs, cats, rabbits, mice, horses, cattle and the like.

"About" refers to a general error range for corresponding values as readily understood by those skilled in the relevant art. In some embodiments, "about" mentioned herein refers to the values described and ranges thereof of ±10%, ±5%, or ±1%.

"EC₅₀", i.e., concentration for 50% of maximal effect, refers to a concentration that causes 50% of maximal effect.

In the present invention, "parent Fc region" may be a naturally occurring Fc region, and a gene encoding the Fc region may be derived from human, mouse, rabbit, camel, and monkey, and preferably human and mouse; for example, the parent Fc region is an Fc region set forth in SEQ ID NO: 77 or 78.

The relevant description of publications mentioned herein is incorporated by reference in its entirety.

### Anti-PD-L1 Antibody

The present invention provides an antibody or an antigen-binding fragment having high affinity for PD-L1 protein. The screened antibodies exhibit potent binding activity and biological activity, and can be used for therapeutic and diagnostic purposes. For example, these antibodies or antigen-binding fragments can effectively block inhibitory immune checkpoints, activate lymphocytes to release cytokines, and can be used for treating various types of cancers, tumors or infections and other related diseases.

In some embodiments, a heavy chain of the antibody comprises a sequence having an amino acid sequence set forth in SEQ ID NO: 77, and a light chain of the antibody comprises a sequence having an amino acid sequence set forth in SEQ ID NO: 92. In some embodiments, a heavy chain of the antigen-binding fragment comprises a sequence having an amino acid sequence set forth in SEQ ID NO: 77 other than Fc, and a light chain of the antigen-binding fragment comprises a sequence having an amino acid sequence set forth in SEQ ID NO: 92.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 93. In some embodiments, the heavy chain of the antigen-binding fragment comprises a sequence having an amino acid sequence set forth in SEQ ID NO: 77 other than Fc, and the light chain of the antigen-binding fragment comprises a sequence having an amino acid sequence set forth in SEQ ID NO: 93.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 94. In some embodiments, the heavy chain of the antigen-binding fragment comprises a sequence having an amino acid sequence set forth in SEQ ID NO: 77 other than Fc, and the light chain of the antigen-binding fragment comprises a sequence having an amino acid sequence set forth in SEQ ID NO: 94.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 95. In some embodiments, the heavy chain of the antigen-binding fragment comprises a sequence having an amino acid sequence set forth in SEQ ID NO: 77 other than Fc, and the light chain of the antigen-binding fragment comprises a sequence having an amino acid sequence set forth in SEQ ID NO: 95.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 96. In some embodiments, the heavy chain of the antigen-binding fragment comprises a sequence having an amino acid sequence set forth in SEQ ID NO: 77 other than Fc, and the light chain of the antigen-binding fragment comprises a sequence having an amino acid sequence set forth in SEQ ID NO: 96.

In some embodiments, the heavy chain of the antibody comprises a sequence having an amino acid sequence set forth in SEQ ID NO: 77, and the light chain of the antibody comprises a sequence having an amino acid sequence set forth in SEQ ID NO: 97. In some embodiments, the heavy chain of the antigen-binding fragment comprises a sequence having an amino acid sequence set forth in SEQ ID NO: 77 other than Fc, and the light chain of the antigen-binding fragment comprises a sequence having an amino acid sequence set forth in SEQ ID NO: 97.

In some embodiments, the heavy chain of the antibody comprises a sequence having an amino acid sequence set forth in SEQ ID NO: 78, and the light chain of the antibody comprises a sequence having an amino acid sequence set forth in SEQ ID NO: 92. In some embodiments, the heavy chain of the antigen-binding fragment comprises a sequence having an amino acid sequence set forth in SEQ ID NO: 78 other than Fc, and the light chain of the antigen-binding fragment comprises a sequence having an amino acid sequence set forth in SEQ ID NO: 92.

In some embodiments, the heavy chain of the antibody comprises a sequence having an amino acid sequence set forth in SEQ ID NO: 79, and the light chain of the antibody comprises a sequence having an amino acid sequence set forth in SEQ ID NO: 92. In some embodiments, the heavy chain of the antigen-binding fragment comprises a sequence having an amino acid sequence set forth in SEQ ID NO: 79 other than Fc, and the light chain of the antigen-binding fragment comprises a sequence having an amino acid sequence set forth in SEQ ID NO: 92.

In some embodiments, the heavy chain of the antibody comprises a sequence having an amino acid sequence set forth in SEQ ID NO: 80, and the light chain of the antibody comprises a sequence having an amino acid sequence set forth in SEQ ID NO: 92. In some embodiments, the heavy chain of the antigen-binding fragment comprises a sequence having an amino acid sequence set forth in SEQ ID NO: 80 other than Fc, and the light chain of the antigen-binding fragment comprises a sequence having an amino acid sequence set forth in SEQ ID NO: 92.

In some embodiments, the heavy chain of the antibody comprises a sequence having an amino acid sequence set forth in SEQ ID NO: 81, and the light chain of the antibody comprises a sequence having an amino acid sequence set forth in SEQ ID NO: 92. In some embodiments, the heavy chain of the antigen-binding fragment comprises a sequence having an amino acid sequence set forth in SEQ ID NO: 81 other than Fc, and the light chain of the antigen-binding fragment comprises a sequence having an amino acid sequence set forth in SEQ ID NO: 92.

In some embodiments, the heavy chain of the antibody comprises a sequence having an amino acid sequence set forth in SEQ ID NO: 82, and the light chain of the antibody comprises a sequence having an amino acid sequence set forth in SEQ ID NO: 92. In some embodiments, the heavy chain of the antigen-binding fragment comprises a sequence having an amino acid sequence set forth in SEQ ID NO: 82 other than Fc, and the light chain of the antigen-binding fragment comprises a sequence having an amino acid sequence set forth in SEQ ID NO: 92.

In some embodiments, the antibody of the present invention comprises two identical heavy chains (or heavy chain fragments) and two identical light chains (or light chain fragments).

It will also be appreciated by those of ordinary skills in the art that the sequence of the antibody or the antigen-binding fragment disclosed herein may be substituted, and the substituted amino acid sequence differs from the naturally occurring amino acid sequence of the antibody. For example, the substituted amino acid sequence can be similar to the starting sequence, for example, having a certain proportion of identity to the starting sequence, for example, the identity to the starting sequence may be about 80%, about 85%, about 90%, about 95%, about 98%, about 99%, or a range between any two of these values (inclusive) or any value therein.

In some embodiments, the antibody or the antigen-binding fragment comprises an amino acid sequence having one or more modification groups. For example, the antibody or the antigen-binding fragment disclosed herein may comprise a flexible linker sequence, or may be modified to add a functional group (e.g., PEG, a drug, a toxin, or a tag).

The antibody or the antigen-binding fragment disclosed herein includes modified derivatives, i.e., modified by covalent linking of any type of molecules to the antibody or the antigen-binding fragment, wherein the covalent linking does not prevent the antibody or the antigen-binding fragment from binding to an epitope. The following examples are included but not by way of limitation, wherein an antibody or an antigen-binding fragment may be modified by glycosylation, acetylation, pegylation, phosphorylation, amidation, derivatization by known protecting/blocking groups, proteolytic cleavage, attachment to cellular ligands or other proteins, or the like. Any of a number of chemical modifications may be made by techniques in the prior art, including but not limited to specific chemical cleavage, acetylation, formylation, metabolic synthesis of tunicamycin, and the like.

In some embodiments, the antibody or the antigen-binding fragment may be conjugated with a therapeutic agent, a prodrug, a peptide, a protein, an enzyme, a virus, a lipid, a biological response modifier, an agent, or PEG.

The antibody or the antigen-binding fragment may be detectably labeled by coupling to a chemiluminescent compound. The presence of the chemiluminescent-labeled antibody or antigen-binding fragment is then determined by detecting the luminescence that occurs during the chemical reaction. Examples of chemiluminescent-labeled compounds include luminol, isoluminol, aromatic acridinium esters, imidazole, acridinium salts and oxalate esters.

### Methods for Preparing Antibodies and Antibody-Encoding Polynucleotides

The present invention further discloses a polynucleotide or a nucleic acid molecule encoding the antibody, the antigen-binding fragment, and a derivative thereof of the present invention. The polynucleotide disclosed herein may encode a heavy chain variable region, a light chain variable region, an Fc region, a portion of a heavy chain variable region, a portion of a light chain variable region, a heavy chain, a light chain, or the like. Methods for preparing antibodies are well known in the art and are described herein.

In certain embodiments, the prepared antibody does not elicit a deleterious immune response in the animal (e.g., human) to be treated. In some embodiments, the antibody, the antigen-binding fragment or the derivative thereof disclosed herein is modified using techniques well known in the art to reduce the immunogenicity. For example, the antibody can be humanized, primatized or deimmunized, or a chimeric antibody can be prepared. Such antibodies are derived from non-human antibodies, typically murine or primate antibodies, which retain or substantially retain the antigen-binding properties of the parent antibody but are less immunogenic in humans. This can be accomplished by a variety of methods, including: (a) grafting an entire variable region of a non-human origin to a constant region of a human origin to produce a chimeric antibody; (b) grafting at least a portion of one or more non-human complementarity determining regions (CDRs) to framework regions and a constant region of a human origin, with or without retention of critical framework residues; or (c) grafting an entire variable region of a non-human origin, but "hiding" the surface residues by replacing them with portions of a human-like origin. Generally, framework residues in the human framework regions will be substituted with corresponding residues from the CDR donor antibody, preferably residues that may improve the antigen binding. Such framework substitutions can be identified by methods well known in the art, for example, by modeling the interaction of the CDR and framework residues to identify framework residues that play an important role in antigen binding and by sequence alignment to identify aberrant framework residues at particular positions (see, U.S. Pat. No. 5,585,089 and Riechmann et al., Nature 332:323 (1988); which are incorporated herein by reference in their entireties). The antibody can be humanized by a variety of techniques well known in the art, such as CDR grafting (Pat. Nos. EP 239,400 and WO 91/09967, and U.S. Pat. Nos. 5,225,539, 5,530,101 and 5,585,089), repair or surface rearrangement (Pat. Nos. EP592,106 and EP519,596; Padlan, et al., Molecular Immunology 28(4/5):489-498 (1991); Studnicka et al., Protein Engineering 7(6):805-814 (1994); Roguska, et al., Proc. Natl. Sci. USA 91:969-973 (1994)), and chain rearrangement (U.S. Pat. No. 5,565,332), which are incorporated herein by reference in their entireties.

Deimmunization may also be used to reduce the immunogenicity of the antibody. In the present invention, the term "deimmunization" includes the alteration of the antibody to modify T cell epitopes (see, e.g., Pat. Nos. WO/9852976 A1 and WO/0034317 A2). For example, a heavy chain variable region sequence and a light chain variable region sequence from the original antibody are analyzed, and a "map" of human T cell epitopes from each variable region is generated, showing the positions of the epitopes relative to the complementarity determining regions (CDRs) and other critical residues within the sequence. Individual T cell epitopes from the T cell epitope map are analyzed to identify alternative amino acid substitutions with a lower risk of altering antibody activity. A series of alternative heavy chain variable region sequences and light chain variable region sequences comprising combinations of amino acid substitutions are designed and subsequently incorporated into a series of binding polypeptides. The genes of intact heavy and light chains comprising the modified variable regions and human constant regions are cloned into an expression vector, and the plasmid is then transferred into a cell line to produce an intact antibody. The antibodies are compared in appropriate biochemical and biological experiments to identify the optimal antibody.

The binding specificity of the antibody or the antigen-binding fragment disclosed herein can be detected by an *in vitro* assay, such as co-immunoprecipitation, radioimmunoassay (RIA) or enzyme-linked immunosorbent assay (ELISA).

An scFv can be prepared by the technique for producing a single chain unit (U.S. Pat. No. 4,694,778; Bird, Science 242:423-442 (1988), Huston et al., Proc. Natl. Acad. Sci. USA 55:5879-5883 (1988), Ward et al., Nature 334:544-554 (1989), and Nie et al., Antibody Therapeutics 3(1):18-62 (2020)). Single chain fusion peptides are produced by amino acid bridging the heavy and light chain fragments of the Fv region to form a single chain unit. The technique for assembling a functional Fv fragment in *E. coli* (Skerra et al., Science 242:1038-1041 (1988)) can also be used.

Examples of techniques that can be used to produce a single chain Fv (scFv) and an antibody include, for example, those described in U.S. Pat. Nos. 4,946,778 and 5,258,498, and Huston et al., Methods in Enzymology, 203:46-88 (1991), Shu et al., Proc. Natl. Sci. USA, 90:1995-1999 (1993) and Skerra et al., Science, 240:1038-1040 (1988). For certain use, including *in vivo* use of antibodies in humans and *in vitro* detection assays, a chimeric antibody, a humanized antibody or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art, see Morrison, Science 229:1202 (1985); Oi et al., BioTechniques 4:214 (1986); Gillies et al., J. Immunol. Methods 125:191-202 (1989); Neuberger et al., Nature 372:604-608 (1984); Takeda et al., Nature 314:452-454 (1985); and U.S. Pat. Nos. 5,807,715, 4,816,567 and 4,816,397, which are incorporated herein by reference in their entireties.

In addition, another efficient method for producing recombinant antibodies, which in particular is capable of producing primate antibodies comprising monkey variable region and human constant region sequences, is disclosed in Newman, Biotechnology 10:1455-1460 (1992), which is incorporated herein by reference in its entirety. Furthermore, this technique is also described in U.S. Pat. Nos. 5,658,570, 5,693,780 and 5,756,096, each of which is incorporated herein by reference in its entirety.

The antibodies can be prepared by a variety of methods known in the art, including phage display methods using antibody libraries from immunoglobulin sequences. Reference may also be made to U.S. Pat. Nos. 4,444,887 and 4,716,111, and PCT Publication Nos. WO98/46645, WO98/50433, WO98/24893, WO98/16654, WO96/34096, WO96/33735 and WO91/10741, each of which is incorporated herein by reference in its entirety.

In another embodiment, DNA encoding the desired monoclonal antibody can be isolated and sequenced using conventional methods (e.g., using oligonucleotide probes that can specifically bind to genes encoding the heavy and light chains of a murine antibody). Isolated and subcloned hybridoma cells can be preferred sources of such DNA. Once isolated, the DNA can be inserted into an expression vector and then transfected into prokaryotic or eukaryotic host cells such as *E. coli* cells, simian COS cells, Chinese hamster ovary (CHO) cells, or myeloma cells that do not produce immunoglobulins. The isolated DNA (which may be synthetic as described herein) can be used to prepare sequences of the constant and variable regions of an antibody, as described in U.S. Pat. No. 5,658,570, which is incorporated herein by reference in its entireties. This method extracts RNA from the selected cells and conversion to cDNA, followed by amplification by PCR using Ig-specific primers. Suitable probes for this purpose are also described in U.S. Pat. No. 5,658,570.

In addition, using conventional recombinant DNA techniques, one or more CDRs of the antibody disclosed herein can be inserted into framework regions, e.g., into human framework regions to construct a humanized non-fully human antibody. The framework regions can be naturally occurring or consensus framework regions, preferably human framework regions (see Chothia et al., J. Mol. Biol. 278:457-479 (1998), in which a range of human framework regions are listed). Some polynucleotides may encode antibodies produced by the framework region and CDR combinations that specifically bind to at least one epitope of an target antigen. One or more amino acid substitutions may be made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

Antibodies can be prepared by using conventional recombinant DNA techniques. Vectors and cell lines for antibody production can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

In some embodiments, the DNA encoding the antibody can be designed and synthesized according to the antibody amino acid sequence described herein by conventional methods, inserted into an expression vector, and transfected into a host cell. The transfected host cell is then cultured in a medium to produce the monoclonal antibody. In some embodiments, the vector expressing the antibody comprises at least one promoter element, an antibody coding sequence, a transcription termination signal and a poly A tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, or Plncx, pcDNA3.1 (+/-), pcDNA/Zeo (+/-), pcDNA3.1/Hygro(+/-), PSVL, PMSG, pRSVcat, pSV2dhfr, pBC12MI, pCS2, etc. Commonly used mammalian cells include 293 cells, Cos1 cells, Cos7 cells, CV1 cells, murine L cells, CHO cells and the like.

In some embodiments, the inserted gene fragment should comprise a screening label, common ones of which include screening genes such as dihydrofolate reductase, glutamine synthetase, neomycin resistance and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein.

In addition, mutations can be introduced in the nucleotide sequence encoding the antibody disclosed herein using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids or a substitution of less than 2 amino acids relative to the original heavy chain variable region and the light chain variable region. Alternatively, mutations can be introduced randomly along all or part of the coding sequence, for example by saturation mutagenesis, and the resulting mutants can be screened for the biological activity to identify mutants that retain the activity.

### Treatment Method

The present invention further provides a treatment method and use. In some embodiments, provided is a method for treating or ameliorating various types of cancers, tumors or infections and other related diseases, wherein the method comprises administering to a patient an effective dose of the anti-PD-L1 antibody or the antigen-binding fragment. In some embodiments, provided is use of the anti-PD-L1 antibody or the antigen-binding fragment in the treatment or amelioration of cancers, tumors or infections and other related diseases. In some embodiments, provided is use of the anti-PD-L1 antibody or the antigen-binding fragment in the manufacture of a medicament for treating or ameliorating cancers, tumors or infections and other related diseases.

The specific dosage and regimen for any particular patient will depend on a variety of factors including the particular antibody or derivative thereof used, the age and body weight of the patient, general health condition, sex and diet, and the time of administration, frequency of excretion, drug combination and the severity of the particular disease being treated. These factors are judged by medical caregivers included within the scope of those of ordinary skills in the art. The dosage will also depend on the individual patient to be treated, the route of administration, the type of the formulation, the nature of the compound used, the severity of the disease and the efficacy desired. The dosage employed can be determined by pharmacological and pharmacokinetic principles well known in the art.

The modes of administration for the antibody or the derivative include, but are not limited to, intradermal, intramuscular, intraperitoneal, intravenous, subcutaneous, nasal, epidural and oral injection. The pharmaceutical composition may be administered by any convenient route, e.g., by infusion or bolus injection, by absorption through epithelial or cutaneous mucosa (e.g., oral mucosa, rectal and intestinal mucosa), and may be co-administered with other biologically active agents. Thus, the pharmaceutical composition comprising the antibody or the antigen-binding fragment disclosed herein can be administered orally, rectally, parenterally, intracisternally, intravaginally, intraperitoneally, topically (e.g. by powder, ointment, drop or transdermal patch) or buccally, or by oral or nasal spray.

The term "parenteral" as used herein refers to modes of administration including intravenous, intramuscular, intraperitoneal, intrasternal, subcutaneous and intraarticular injections and infusions.

The mode of administration may be systemic or local. In addition, it may be desirable to introduce the antibody disclosed herein into the central nervous system by any suitable route, including intraventricular and intrathecal injections; intraventricular injection may be assisted by an intraventricular catheter connected to, for example, a reservoir (which may be an Ommaya reservoir). Pulmonary administration is also possible, for example by use of an inhaler or nebulizer, and by use of nebulized formulations.

The antibodies disclosed herein may be administered locally to an area in need of treatment, which may be done by, but not limited to, the following: local infusion during surgery, for example, topical application in combination with a post-operative wound dressing, achieved by injection, a catheter, a suppository or an implant which is a porous, non-porous or gelatinous material, including membranes (e.g. silicone rubber membranes) or fibers. Preferably, when the protein disclosed herein (including antibodies) is administered, care must be taken to use materials that do not absorb the protein.

In some embodiments, the composition disclosed herein comprises a nucleic acid or a polynucleotide encoding an antibody, which can be administered *in vivo* to facilitate expression of the encoded protein by constructing the nucleic acid or the polynucleotide as part of a suitable nucleic acid expression vector; and a part of the vector is administered to be intracellular by the following methods, for example, by using a retroviral vector (see U.S. Pat. No. 4,980,286), by direct injection, by using microprojectile bombardment (e.g., gene gun; Biolistic, Dupont), by coating with lipids or cell surface receptors or transfection reagents, or by ligation with homeobox-like peptides with known capability of entering the nucleus (see, e.g., Joliot et al., 1991, Proc. Natl. Acad. Sci. USA 88:1864-1868). Alternatively, the nucleic acid can be introduced into the cell and integrated into the host cell DNA for expression by homologous recombination.

In some embodiments, the antibody of the present invention is administered to a patient at a dose of 0.01 mg/kg to 100 mg/kg of patient body weight, or 0.1 mg/kg to 20 mg/kg of patient body weight. A second or more doses of the antibody or the antigen-binding fragment may be administered subsequently to the initial dose, at about the same or less dose as the initial dose, wherein the administration at the subsequent dose may be separated by at least 1 to 3 days, or at least one week. The dose and frequency for administration of the antibody disclosed herein can be reduced by enhancing the uptake and tissue penetration ability (e.g., into the brain) of the antibody through modifications such as lipidation.

Various known delivery systems can be used to administer the antibody or the derivative, or the polynucleotide encoding the same disclosed herein, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), construction of nucleic acids as part of a retrovirus or other vectors.

### Combination Therapy

In some embodiments, the anti-PD-L1 antibody or the antigen-binding fragment of the present invention can be administered in combination with other therapeutic or prophylactic regimens, including administration of one or more antibodies or antigen-binding fragments of the present invention together or in combination with one or more other therapeutic agents or methods. In some embodiments, other therapeutic regimens include, but are not limited to, radiation therapy, chemotherapy, hormone therapy, and the like. For combination therapy, the antibody may be administered simultaneously or separately with other therapeutic agents. When administered separately, the antibody disclosed herein can be administered before or after administration of another additional therapeutic agent.

In some embodiments, the antibody disclosed herein is administered in combination with a chemotherapeutic agent. In some embodiments, chemotherapeutic agents that can be administered with the antibody disclosed herein include, but are not limited to, antibiotic derivatives (e.g., doxorubicin, bleomycin, daunorubicin and actinomycin D), antiestrogens (e.g., tamoxifen), antimetabolites (e.g., fluorouracil, 5-FU, methotrexate, floxuridine, interferon α-2b, glutamic acid, mithramycin, mercaptopurine and 6-thioguanine), cytotoxic agents (e.g., carmustine, BCNU, lomustine, CCNU, cytarabine, cyclophosphamide, estramustine, hydroxyurea, procarbazine, mitomycin, busulfan, cisplatin and vincristine sulfate), hormones (e.g., medroxyprogesterone, estramustine sodium phosphate, ethinylestradiol, estradiol, megestrol acetate, methyltestosterone, stilbestrol diphosphate, chlorotrianisene and testolactone), nitrogen mustard derivatives (e.g., melphalan, chlorambucil, dichloromethyl diethylamine (mechlorethamine) and thiotepa), steroids and combinations thereof (e.g., betamethasone sodium phosphate), and other compounds (e.g., dacarbazine, asparaginase, mitotane, vincristine sulfate, vinblastine sulfate and etoposide). In some embodiments, the antibody disclosed herein is administered in combination with cytokines. Cytokines that can be administered with the antibody disclosed herein include, but are not limited to, IL-2, IL-3, IL-4, IL-5, IL-6, IL-7, IL-10, IL-12, IL-13, IL-15, and the like.

In some embodiments, the anti-PD-L1 antibody disclosed herein is administered in combination with a chemotherapeutic agent. Examples of the chemotherapeutic agents include immunotherapeutic agents, including but not limited to therapeutic antibodies suitable for treating a patient. Some examples of the therapeutic antibodies include rituximab, trastuzumab, tositumomab, ibritumomab, alemtuzumab, epratuzumab, bevacizumab, cetuximab, brentuximab, and the like.

### Pharmaceutical Composition

The present invention further provides a pharmaceutical composition. Such compositions comprise an effective dose of an anti-PD-L1 antibody or an antigen-binding fragment and a pharmaceutically acceptable excipient. In some embodiments, the pharmaceutical composition comprises 0.1%-90% of the anti-PD-L1 antibody or the antigen-binding fragment. In some embodiments, the pharmaceutical composition further comprises an anti-cancer agent (e.g., an immune checkpoint inhibitor).

In some embodiments, the term "pharmaceutically acceptable" refers to a substance approved by a government regulatory agency or listed in commonly-recognized pharmacopeias for use in animals, and particularly in humans. In addition, the "pharmaceutically acceptable excipient" generally may be any type of non-toxic solid, semi-solid or liquid filler, diluent, an encapsulating material, or a formulation auxiliary.

The term "excipient" refers to a diluent, adjuvant, excipient or carrier that can be administered to a patient together with the active ingredient. Such pharmaceutical carriers can be sterile liquids, such as water and oils, including petroleum, oils originated from animal, plant or synthesis, such as peanut oil, soybean oil, mineral oil, sesame oil, and the like. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol and the like. If desired, the composition may also comprise a small amount of a wetting agent, an emulsifier, or a pH buffering agent such as acetates, citrates or phosphates. Antibacterials such as benzyl alcohol or methylparaben, antioxidants such as ascorbic acid or sodium bisulfite, chelating agents such as ethylenediamine tetraacetic acid, and tonicity adjusting agents such as sodium chloride or dextrose are also contemplated. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or an antigen-binding fragment, preferably in purified form, together with an appropriate amount of an excipient, to provide a form suitable for administration to a patient. The formulation should be suitable for the administration mode. The parental formulation may be enclosed in ampoules, disposable syringes or multiple dose vials made of glass or plastic.

In some embodiments, the composition is formulated into a pharmaceutical composition suitable for intravenous injection into a human body according to conventional steps. A composition for intravenous administration is usually a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture, for example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administrated by injection, an ampoule bottle containing sterile water or saline for injection can be used, so that the active ingredients can be mixed before administration.

The compound disclosed herein may be formulated in a neutral or salt form. Pharmaceutically acceptable salts include salts formed with anions derived from acids such as hydrochloric acid, phosphoric acid, acetic acid, oxalic acid and tartaric acid, and salts formed with cations derived from, e.g., sodium, potassium, ammonium, calcium or iron hydroxide, isopropylamine, triethylamine, 2-ethylaminoethanol, histidine, and procaine.

### DETAILED DESCRIPTION

The technical schemes of the present invention will be further illustrated below through specific examples, which are not intended to limit the protection scope of the present invention. Some nonessential modifications and adjustments made by other people according to the concept of the present invention still fall within the protection scope of the present invention.

The materials, reagents, and the like used in the following examples can be commercially available unless otherwise stated.

### Anti-PD-L1 Antibody

### Example 1. Preparation Method for Antibodies

The amino acids and nucleic acid sequences related to the antibody examples are shown in Tables 1-13, and the heavy chain and light chain compositions of the antibody are shown in Table 1; the Fc regions of the heavy chains in Table 11 are underlined, and the nucleic acid sequences of the variable regions in Table 13 are underlined.

Sequence optimization was performed on the amino acid sequences of the heavy chain and the light chain of the antibody according to codon usage bias of the host cell to obtain DNA sequences of the heavy chain and the light chain. To facilitate expression in the host cell, signal peptides were added to the sequences of the heavy chain and the light chain, respectively.

The optimized and synthesized sequence clones were cloned into a vector (the vector could be pCDNA3.1^{™}(+), Invitrogen, Cat. No. V79020), then a large number of plasmids were extracted, and the heavy chain and the light chain were transiently transfected into HEK293F cells according to the plasmid molar ratio of 1:1. Taking Antibody L1-R2-4-46 as an example, the heavy chain nucleic acid sequence (SEQ ID NO: 99) and the light chain nucleic acid sequence (SEQ ID NO: 101) were cloned into an expression vector by enzyme digestion; wherein the nucleic acid sequence of the heavy chain signal peptide was set forth in SEQ ID NO: 102, and the nucleic acid sequence of the light chain signal peptide was set forth in SEQ ID NO: 103. Taking Antibody L1-R2-4-71 as an example, the heavy chain nucleic acid sequence (SEQ ID NO: 100) and the light chain nucleic acid sequence (SEQ ID NO: 101) were cloned into an expression vector by enzyme digestion; wherein the nucleic acid sequence of the heavy chain signal peptide was set forth in SEQ ID NO: 102, and the nucleic acid sequence of the light chain signal peptide was set forth in SEQ ID NO: 103. After cell expression, the culture medium was purified by immobilized metal affinity chromatography (IMAC) through a Protein A column (GE Healthcare) to purify the secreted antibody protein containing two heavy chains and two light chains, so that the purity of the antibody was more than 95%. The sequencing result was the same as the expected sequence, and the antibody was used for affinity assay and biological activity identification.

**Table 1. Composition of antibodies**

| Antibody No. | Heavy chain SEQ ID NO (sequence No.) | Light chain sequence No. |
|---|---|---|
| L1-R2-4 | 77 | 92 |
| L1-R2-6 | 77 | 93 |
| L1-R2-18 | 77 | 94 |
| L1-R2-78 | 77 | 95 |
| L1-R2-85 | 77 | 96 |
| L1-R2-89 | 77 | 97 |
| L1-R2-4-36 | 78 | 92 |
| L1-R2-4-46 | 79 | 92 |
| L1-R2-4-47 | 80 | 92 |
| L1-R2-4-55 | 81 | 92 |
| L1-R2-4-71 | 82 | 92 |

**Table 2. Heavy chain CDRs of antibodies**

| Amino acid sequence of HCDR1 | Sequence No. | Amino acid sequence of HCDR2 | Sequence No. | Amino acid sequence of HCDR3 | Sequence No. |
|---|---|---|---|---|---|
| GFTFSDSWIH | 1 | GWISPYGGSTYYADDFRH | 7 | RHWPGGFDY | 17 |
| SFSLKDSWIH | 2 | GWISPYGGSTYYADSLGD | 8 | RHWPGGLLP | 18 |
| GWATRDSWIH | 3 | GWISPYGGSTYYADHLSQ | 9 | / | |
| NSHLRDSWIH | 4 | GWISPYGGSTYYADGMRS | 10 | | |
| GLRPADSWIH | 5 | GWISPYGGSTYYADSYRS | 11 | | |
| GFTFSDSWIH | 6 | GWISPYGGSTYYADPYVG | 12 | | |
| / | GWISPYGGSTYYADSLKG | | | 13 | |
| | GWISPYGGSTYYADSYRA | | | 14 | |
| | GWISPYGGSTYYADYLHG | | | 15 | |
| | GWISPYGGSTYYADSVKG | | | 16 | |

**Table 3. Composition of heavy chain CDRs of antibodies**

| Name | Sequence number of HCDR1 | Sequence number of HCDR2 | Sequence number of HCDR3 |
|---|---|---|---|
| CP11-36 | 1 | 7 | 17 |
| CP11-46 | 1 | 8 | 17 |
| CP11-47 | 1 | 9 | 17 |
| CP11-55 | 1 | 10 | 17 |
| CP11-71 | 1 | 11 | 17 |
| CP14-16 | 1 | 12 | 17 |
| CP14-80 | 1 | 13 | 17 |
| CP21-8 | 1 | 14 | 17 |
| CP21-47 | 1 | 15 | 17 |
| CP21-59 | 2 | 16 | 17 |
| CP22-34 | 3 | 16 | 17 |
| CP22-45 | 4 | 16 | 17 |
| CP22-44 | 5 | 16 | 17 |
| CP22-40 | 6 | 16 | 18 |
| L1-27 | 1 | 16 | 17 |

**Table 4. Light chain CDRs of antibodies**

| Amino acid sequence | Sequence No. | Amino acid sequence | Sequence No. | Amino acid sequence | Sequence No. |
|---|---|---|---|---|---|
| of LCDR1 | | of LCDR2 | | of LCDR3 | |
| LASQTIGTWLA | 19 | AASTLQS | 24 | QQYYSTPRT | 28 |
| QASQDIGKHLN | 20 | GASNLKT | 25 | QQSYTTPYT | 29 |
| RASQGIGSWLA | 21 | AASSLQS | 26 | QQYFSTPYT | 30 |
| RASQGISSYLA | 22 | AASRLQS | 27 | QQYYSTPYT | 31 |
| RASQGIGKFLA | 23 | / | | | |

**Table 5. Composition of light chain CDRs of antibodies**

| Name | Sequence number of LCDR1 | Sequence number of LCDR2 | Sequence number of LCDR3 |
|---|---|---|---|
| R2-4 | 19 | 24 | 28 |
| R2-6 | 20 | 25 | 29 |
| R2-18 | 21 | 26 | 30 |
| R2-78 | 22 | 27 | 31 |
| R2-85 | 23 | 26 | 30 |
| R2-89 | 19 | 26 | 31 |

**Table 6. Light chain framework regions of antibodies**

| Name | Amino acid sequence | Sequence No. | Name | Amino acid sequence | Sequence No. |
|---|---|---|---|---|---|
| FR1 | EIVLTQSPSSLSASVGDRVTITC | 51 | FR2 | WYQQKPGKSPQLLIY | 57 |
| | EIVLTQSPSSVSASVGDKLTMTC | 52 | | WYQQKPGKPPKLLIY | 58 |
| | EIVLTQSPSSVSASVGDRITITC | 53 | | WYQQKPGKAPSLLIY | 59 |
| | DIQMTQSPSSFSASTGDRVTITC | 54 | | WYQQKPGKAPKLLI Y | 60 |
| | DIQMTQSPSSLSASVGDRVTIPC | 55 | | / | |
| | DIQMTQSPSSLSASVGDRVTITC | 56 | | | |
| FR3 | GVPSRFSGSGSGTDFTLTISSLQPEDVATYYC | 61 | FR4 | FGQGTKVEIK | 65 |
| | GVPSRFSGTGSGTDFTLTISSLQPEDFATYYC | 62 | | FGQGTRLEIK | 66 |
| | GVPSRFSGSGSGTDFTLTISSLQTEDVAVYYC | 63 | | FGQGTKVDIK | 67 |
| | GVPSRFSGSGSGTDFTLTISSLQAEDVAVYY C | 64 | | / | |

**Table 7. Heavy chain framework regions of antibodies**

| Name | Amino acid sequence | Sequence No. | Name | Amino acid sequence | Sequence No. |
|---|---|---|---|---|---|
| FR1 | EVQLVESGGGLVQPGGSLRLSCAAS | 32 | FR2 | WVRQAPGKGLEWV | 33 |
| FR3 | | 34 | FR4 | WGQGTLVTVSS | 35 |

**Table 8. Heavy chain variable regions of antibodies**

| Name | Amino acid sequence | Sequence No. |
|---|---|---|
| L1-27 | EVQLVESGGGLVQPGGSLRLSCAASGFTFSDSWIHWVRQAPGKGLEWV GWISPYGGSTYYADSVKGRFTISADTSKNTAYLQMNSLRAEDTAVYYC ARRHWPGGFDYWGQGTLVTVSS | 36 |
| CP11-36 | EVQLVESGGGLVQPGGSLRLSCAASGFTFSDSWIHWVRQAPGKGLEWV GWISPYGGSTYYADDFRHRFTISADTSKNTAYLQMNSLRAEDTAVYYC ARRHWPGGFDYWGQGTLVTVSS | 37 |
| CP11-46 | EVQLVESGGGLVQPGGSLRLSCAASGFTFSDSWIHWVRQAPGKGLEWV GWISPYGGSTYYADSLGDRFTISADTSKNTAYLQMNSLRAEDTAVYYC ARRHWPGGFDYWGQGTLVTVSS | 38 |
| CP11-47 | EVQLVESGGGLVQPGGSLRLSCAASGFTFSDSWIHWVRQAPGKGLEWV GWISPYGGSTYYADHLSQRFTISADTSKNTAYLQMNSLRAEDTAVYYC ARRHWPGGFDYWGQGTLVTVSS | 39 |
| CP11-55 | EVQLVESGGGLVQPGGSLRLSCAASGFTFSDSWIHWVRQAPGKGLEWV GWISPYGGSTYYADGMRSRFTISADTSKNTAYLQMNSLRAEDTAVYYC ARRHWPGGFDYWGQGTLVTVSS | 40 |
| CP11-71 | EVQLVESGGGLVQPGGSLRLSCAASGFTFSDSWIHWVRQAPGKGLEWV GWISPYGGSTYYADSYRSRFTISADTSKNTAYLQMNSLRAEDTAVYYCA RRHWPGGFDYWGQGTLVTVSS | 41 |
| CP14-16 | EVQLVESGGGLVQPGGSLRLSCAASGFTFSDSWIHWVRQAPGKGLEWV GWISPYGGSTYYADPYVGRFTISADTSKNTAYLQMNSLRAEDTAVYYC ARRHWPGGFDYWGQGTLVTVSS | 42 |
| CP14-80 | EVQLVESGGGLVQPGGSLRLSCAASGFTFSDSWIHWVRQAPGKGLEWV GWISPYGGSTYYADSLKGRFTISADTSKNTAYLQMNSLRAEDTAVYYC ARRHWPGGFDYWGQGTLVTVSS | 43 |
| CP21-8 | EVQLVESGGGLVQPGGSLRLSCAASGFTFSDSWIHWVRQAPGKGLEWV GWISPYGGSTYYADSYRARFTISADTSKNTAYLQMNSLRAEDTAVYYC ARRHWPGGFDYWGQGTLVTVSS | 44 |
| CP21-47 | EVQLVESGGGLVQPGGSLRLSCAASGFTFSDSWIHWVRQAPGKGLEWV GWISPYGGSTYYADYLHGRFTISADTSKNTAYLQMNSLRAEDTAVYYC ARRHWPGGFDYWGQGTLVTVSS | 45 |
| CP21-59 | EVQLVESGGGLVQPGGSLRLSCAASSFSLKDSWIHWVRQAPGKGLEWV GWISPYGGSTYYADSVKGRFTISADTSKNTAYLQMNSLRAEDTAVYYC ARRHWPGGFDYWGQGTLVTVSS | 46 |
| CP22-34 | EVQLVESGGGLVQPGGSLRLSCAASGWATRDSWIHWVRQAPGKGLEW VGWISPYGGSTYYADSVKGRFTISADTSKNTAYLQMNSLRAEDTAVYY CARRHWPGGFDYWGQGTLVTVSS | 47 |
| CP22-40 | EVQLVESGGGLVQPGGSLRLSCAASGFTFSDSWIHWVRQAPGKGLEWV GWISPYGGSTYYADSVKGRFTISADTSKNTAYLQMNSLRAEDTAVYYC ARRHWPGGLLPWGQGTLVTVSS | 48 |
| CP22-44 | EVQLVESGGGLVQPGGSLRLSCAASGLRPADSWIHWVRQAPGKGLEW VGWISPYGGSTYYADSVKGRFTISADTSKNTAYLQMNSLRAEDTAVYY CARRHWPGGFDYWGQGTLVTVSS | 49 |
| CP22-45 | EVQLVESGGGLVQPGGSLRLSCAASNSHLRDSWIHWVRQAPGKGLEW VGWISPYGGSTYYADSVKGRFTISADTSKNTAYLQMNSLRAEDTAVYY CARRHWPGGFDYWGQGTLVTVSS | 50 |

**Table 9. Light chain variable regions of antibodies**

| Name | Amino acid sequence | Sequence No. |
|---|---|---|
| R2-4 | EIVLTQSPSSLSASVGDRVTITCLASQTIGTWLAWYQQKPGKSPQLLIYAA STLQSGVPSRFSGSGSGTDFTLTISSLQPEDVATYYCQQYYSTPRTFGQGT KVEIK | 68 |
| R2-6 | EIVLTQSPSSVSASVGDKLTMTCQASQDIGKHLNWYQQKPGKPPKLLIYG ASNLKTGVPSRFSGTGSGTDFTLTISSLQPEDFATYYCQQSYTTPYTFGQG TRLEIK | 69 |
| R2-18 | EIVLTQSPSSVSASVGDRITITCRASQGIGSWLAWYQQKPGKAPSLLIYAA SSLQSGVPSRFSGSGSGTDFTLTISSLQTEDVAVYYCQQYFSTPYTFGQGT KVDIK | 70 |
| R2-78 | DIQMTQSPSSFSASTGDRVTITCRASQGISSYLAWYQQKPGKAPKLLIYAA SRLQSGVPSRFSGSGSGTDFTLTISSLQAEDVAVYYCQQYYSTPYTFGQGT KVDIK | 71 |
| R2-85 | DIQMTQSPSSLSASVGDRVTIPCRASQGIGKFLAWYQQKPGKAPSLLIYAA SSLQSGVPSRFSGSGSGTDFTLTISSLQTEDVAVYYCQQYFSTPYTFGQGT KVDIK | 72 |
| R2-89 | DIQMTQSPSSLSASVGDRVTITCLASQTIGTWLAWYQQKPGKSPQLLIYA ASSLQSGVPSRFSGSGSGTDFTLTISSLQPEDVATYYCQQYYSTPYTFGQG TKVDIK | 73 |

**Table 10. Constant regions of antibodies**

| Name | Amino acid sequence | Sequence No. |
|---|---|---|
| Heavy chain constant region | | 74 |
| | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEV TCVVVDVSHEDPEVKFNWYVDGVEVHNAKTKPREEQYASTYRVV SVLTVLHQDWLNGKEYKCKVSNKALPAPIEKTISKAKGQPREPQVY TLPPSREEMTKNQVSLTCLVKGFYPSDIAVEWESNGQPENNYKTTPP VLDSDGSFFLYSKLTVDKSRWQQGNVFSCSVMHEALHNHYTQKSL SLSPGK | 75 |
| Light chain constant region | | 76 |

**Table 11. Heavy chain sequences of antibodies**

| Name | Amino acid sequence | Sequence No. |
|---|---|---|
| L1-27 | EVQLVESGGGLVQPGGSLRLSCAASGFTFSDSWIHWVRQAPGKGLEW VGWISPYGGSTYYADSVKGRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI | 77 |
| Heavy chain | | |
| | SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | |
| CP11-36 | EVQLVESGGGLVQPGGSLRLSCAASGFTFSDSWIHWVRQAPGKGLEW VGWISPYGGSTYYADDFRHRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 78 |
| CP11-46 | EVQLVESGGGLVQPGGSLRLSCAASGFTFSDSWIHWVRQAPGKGLEW VGWISPYGGSTYYADSLGDRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 79 |
| CP11-47 | EVQLVESGGGLVQPGGSLRLSCAASGFTFSDSWIHWVRQAPGKGLEW VGWISPYGGSTYYADHLSQRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 80 |
| CP11-55 | EVQLVESGGGLVQPGGSLRLSCAASGFTFSDSWIHWVRQAPGKGLEW VGWISPYGGSTYYADGMRSRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG | 81 |
| | QPENNYKTTPPVLDSDGSFFLYSKLTVDKSRWQQGNVFSCSVMHEALH NHYTQKSLSLSPGK | |
| CP11-71 | EVQLVESGGGLVQPGGSLRLSCAASGFTFSDSWIHWVRQAPGKGLEW VGWISPYGGSTYYADSYRSRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 82 |
| CP14-16 | EVQLVESGGGLVQPGGSLRLSCAASGFTFSDSWIHWVRQAPGKGLEW VGWISPYGGSTYYADPYVGRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 83 |
| CP14-80 | EVQLVESGGGLVQPGGSLRLSCAASGFTFSDSWIHWVRQAPGKGLEW VGWISPYGGSTYYADSLKGRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 84 |
| CP21-8 | EVQLVESGGGLVQPGGSLRLSCAASGFTFSDSWIHWVRQAPGKGLEW VGWISPYGGSTYYADSYRARFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 85 |
| CP21-47 | EVQLVESGGGLVQPGGSLRLSCAASGFTFSDSWIHWVRQAPGKGLEW VGWISPYGGSTYYADYLHGRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 86 |
| CP21-59 | EVQLVESGGGLVQPGGSLRLSCAASSFSLKDSWIHWVRQAPGKGLEW VGWISPYGGSTYYADSVKGRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 87 |
| CP22-34 | EVQLVESGGGLVQPGGSLRLSCAASGWATRDSWIHWVRQAPGKGLEW VGWISPYGGSTYYADSVKGRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 88 |
| CP22-40 | EVQLVESGGGLVQPGGSLRLSCAASGFTFSDSWIHWVRQAPGKGLEW VGWISPYGGSTYYADSVKGRFTISADTSKNTAYLQMNSLRAEDTAVYY CARRHWPGGLLPWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 89 |
| CP22-44 | EVQLVESGGGLVQPGGSLRLSCAASGLRPADSWIHWVRQAPGKGLEW VGWISPYGGSTYYADSVKGRFTISADTSKNTAYLQMNSLRAEDTAVYY | 90 |
| | CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | |
| CP22-45 | EVQLVESGGGLVQPGGSLRLSCAASNSHLRDSWIHWVRQAPGKGLEW VGWISPYGGSTYYADSVKGRFTISADTSKNTAYLQMNSLRAEDTAVYY CARRHWPGGFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK | 91 |
| Heavy chain signal peptide | MEFGLSWVFLVAILKGVQC | 104 |

**Table 12. Light chain sequences of antibodies**

| Name | Amino acid sequence | Sequence |
|---|---|---|
| R2-4 light chain | EIVLTQSPSSLSASVGDRVTITCLASQTIGTWLAWYQQKPGKSPQLLIYAASTLQ SGVPSRFSGSGSGTDFTLTISSLQPEDVATYYCQQYYSTPRTFGQGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC | 92 |
| R2-6 light chain | EIVLTQSPSSVSASVGDKLTMTCQASQDIGKHLNWYQQKPGKPPKLLIYGASNL KTGVPSRFSGTGSGTDFTLTISSLQPEDFATYYCQQSYTTPYTFGQGTRLEIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 93 |
| R2-18 light chain | EIVLTQSPSSVSASVGDRITITCRASQGIGSWLAWYQQKPGKAPSLLIYAASSLQS GVPSRFSGSGSGTDFTLTISSLQTEDVAVYYCQQYFSTPYTFGQGTKVDIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC | 94 |
| R2-78 light chain | DIQMTQSPSSFSASTGDRVTITCRASQGISSYLAWYQQKPGKAPKLLIYAASRLQ SGVPSRFSGSGSGTDFTLTISSLQAEDVAVYYCQQYYSTPYTFGQGTKVDIKRTV | 95 |
| | AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | |
| R2-85 light chain | DIQMTQSPSSLSASVGDRVTIPCRASQGIGKFLAWYQQKPGKAPSLLIYAASSLQ SGVPSRFSGSGSGTDFTLTISSLQTEDVAVYYCQQYFSTPYTFGQGTKVDIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 96 |
| R2-89 light chain | DIQMTQSPSSLSASVGDRVTITCLASQTIGTWLAWYQQKPGKSPQLLIYAASSLQ SGVPSRFSGSGSGTDFTLTISSLQPEDVATYYCQQYYSTPYTFGQGTKVDIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 97 |
| Light chain signal peptide | MDMRVLAQLLGLLLLCFPGARC | 105 |

**Table 13. Related nucleic acid sequences of antibodies**

| Name | Nucleic acid sequence | Sequence |
|---|---|---|
| CP11-46 heavy chain | GAGGTGCAGCTGGTGGAATCCGGCGGAGGGCTGGTGCAGCCAGGTGGC TCTCTGAGACTGAGTTGCGCCGCTTCAGGATTCACCTTTTCCGACTCTTG GATTCACTGGGTGCGCCAGGCCCCCGGAAAGGGACTGGAGTGGGTCGG TTGGATTTCTCCTTACGGAGGGAGTACATACTATGCCGACAGTCTTGGTG ATCGATTCACTATCTCCGCTGATACTAGCAAAAACACCGCATATCTGCAG ATGAACTCCCTGAGGGCAGAAGACACAGCCGTCTACTATTGTGCAAGGC GGCATTGGCCCGGTGGCTTTGATTACTGGGGTCAGGGTACCCTGGTTAC CGTTAGCAGCGCGAGCACCAAAGGCCCGAGCGTGTTTCCGCTGGCCCC GAGCAGCAAAAGCACCAGCGGTGGCACCGCAGCGCTGGGTTGCCTGGT GAAAGATTATTTCCCGGAACCGGTGACGGTGTCGTGGAACTCAGGCGC CCTGACCAGCGGCGTGCACACCTTCCCGGCTGTCCTACAGTCCTCAGGA CTCTACTCCCTCAGCAGCGTGGTGACCGTGCCCTCCAGCAGCTTGGGCA CCCAGACCTACATCTGCAACGTGAATCACAAGCCCAGCAACACCAAGG TGGACAAGAAAGTTGAGCCCAAATCTTGTGACAAAACTCACACATGCC CACCGTGCCCAGCACCTGAACTCCTGGGGGGACCGTCAGTCTTCCTCTT CCCCCCAAAACCCAAGGACACCCTCATGATCTCCCGGACCCCTGAGGTC ACATGCGTGGTGGTGGACGTGAGCCACGAAGACCCTGAGGTCAAGTTC AACTGGTACGTGGACGGCGTGGAGGTGCATAATGCCAAGACAAAGCCG CGGGAGGAGCAGTACAACAGCACGTACCGGGTGGTCAGCGTCCTCACC GTCCTGCACCAGGACTGGCTGAATGGCAAGGAGTACAAGTGCAAGGTC TCCAACAAAGCCCTCCCAGCCCCCATCGAGAAAACCATCTCCAAAGCC AAAGGGCAGCCCCGAGAACCACAGGTGTACACCCTGCCCCCATCCCGG GATGAGCTGACCAAGAACCAGGTCAGCCTGACCTGCCTGGTCAAAGGC TTCTATCCCAGCGACATCGCCGTGGAGTGGGAGAGCAATGGGCAGCCG | 99 |
| | GAGAACAACTACAAGACCACGCCTCCCGTGCTGGACTCCGACGGCTCC TTCTTCCTCTACAGCAAGCTCACCGTGGACAAGAGCAGGTGGCAGCAG GGGAACGTCTTCTCATGCTCCGTGATGCATGAGGCTCTGCACAACCACT ACACGCAGAAGAGCCTCTCCCTGTCTCCGGGTAAA | |
| CP11-71 heavy chain | GAGGTGCAGCTGGTGGAATCCGGCGGAGGGCTGGTGCAGCCAGGTGGC TCTCTGAGACTGAGTTGCGCCGCTTCAGGATTCACCTTTTCCGACTCTTG GATTCACTGGGTGCGCCAGGCCCCCGGAAAGGGACTGGAGTGGGTCGG TTGGATTTCTCCTTACGGAGGGAGTACATACTATGCCGACTCTTATAGGT CGCGATTCACTATCTCCGCTGATACTAGCAAAAACACCGCATATCTGCAG ATGAACTCCCTGAGGGCAGAAGACACAGCCGTCTACTATTGTGCAAGGC GGCATTGGCCCGGTGGCTTTGATTACTGGGGTCAGGGTACCCTGGTTAC CGTTAGCAGCGCGAGCACCAAAGGCCCGAGCGTGTTTCCGCTGGCCCC GAGCAGCAAAAGCACCAGCGGTGGCACCGCAGCGCTGGGTTGCCTGGT GAAAGATTATTTCCCGGAACCGGTGACGGTGTCGTGGAACTCAGGCGC CCTGACCAGCGGCGTGCACACCTTCCCGGCTGTCCTACAGTCCTCAGGA CTCTACTCCCTCAGCAGCGTGGTGACCGTGCCCTCCAGCAGCTTGGGCA CCCAGACCTACATCTGCAACGTGAATCACAAGCCCAGCAACACCAAGG TGGACAAGAAAGTTGAGCCCAAATCTTGTGACAAAACTCACACATGCC CACCGTGCCCAGCACCTGAACTCCTGGGGGGACCGTCAGTCTTCCTCTT CCCCCCAAAACCCAAGGACACCCTCATGATCTCCCGGACCCCTGAGGTC ACATGCGTGGTGGTGGACGTGAGCCACGAAGACCCTGAGGTCAAGTTC AACTGGTACGTGGACGGCGTGGAGGTGCATAATGCCAAGACAAAGCCG CGGGAGGAGCAGTACAACAGCACGTACCGGGTGGTCAGCGTCCTCACC GTCCTGCACCAGGACTGGCTGAATGGCAAGGAGTACAAGTGCAAGGTC TCCAACAAAGCCCTCCCAGCCCCCATCGAGAAAACCATCTCCAAAGCC AAAGGGCAGCCCCGAGAACCACAGGTGTACACCCTGCCCCCATCCCGG GATGAGCTGACCAAGAACCAGGTCAGCCTGACCTGCCTGGTCAAAGGC TTCTATCCCAGCGACATCGCCGTGGAGTGGGAGAGCAATGGGCAGCCG GAGAACAACTACAAGACCACGCCTCCCGTGCTGGACTCCGACGGCTCC TTCTTCCTCTACAGCAAGCTCACCGTGGACAAGAGCAGGTGGCAGCAG GGGAACGTCTTCTCATGCTCCGTGATGCATGAGGCTCTGCACAACCACT ACACGCAGAAGAGCCTCTCCCTGTCTCCGGGTAAA | 100 |
| R2-4 light chain | GAAATTGTGCTGACTCAGTCTCCATCCTCCCTGTCTGCATCTGTAGGAGA CAGAGTCACCATCACTTGCCTGGCAAGTCAGACCATTGGTACATGGTTA GCATGGTATCAGCAGAAACCAGGGAAATCTCCTCAGCTCCTGATCTATG CTGCATCCACTTTGCAATCAGGGGTCCCATCTCGGTTCAGTGGCAGTGG ATCTGGGACAGATTTCACTCTCACCATCAGCAGCCTGCAGCCTGAAGAT GTTGCAACTTATTACTGTCAGCAATATTATAGTACTCCTCGGACGTTCGG CCAAGGGACCAAGGTGGAGATTAAACGTACGGTGGCTGCACCATCTGT CTTCATCTTCCCGCCATCTGATGAGCAGTTGAAATCTGGAACTGCCTCTG TTGTGTGCCTGCTGAATAACTTCTATCCCAGAGAGGCCAAAGTACAGTG GAAGGTGGATAACGCCCTCCAATCGGGTAACTCCCAGGAGAGTGTCAC AGAGCAGGACAGCAAGGACAGCACCTACAGCCTCAGCAGCACCCTGA | 101 |
| | CGCTGAGCAAAGCAGACTACGAGAAACACAAAGTCTACGCCTGCGAAG TCACCCATCAGGGCCTGAGCTCGCCCGTCACAAAGAGCTTCAACAGGG GAGAGTGT | |
| Heavy chain signal peptide | ATGGAGTTTGGGCTGAGCTGGGTTTTCCTTGTTGCTATATTAAAAGGTGT CCAGTGT | 102 |
| Light chain signal peptide | ATGGACATGAGGGTGCTGGCCCAGCTGCTGGGACTCCTCCTGCTGTGCT TCCCAGGCGCCAGATGC | 103 |

### Example 2. Dissociation Constant Assay

The equilibrium dissociation constant (KD) of the above-described antibodies of the present invention binding to the corresponding antigens was determined by a kinetic binding assay using a Biacore system (GE). According to the method in the manual, 5 µg of the antigen protein PD-L1-His was immobilized on the chip, the affinity assay was performed with the anti-PD-L1 antibody at the highest concentration of 100 nM (diluted in a ratio of 1:2, 5 gradients) as the mobile phase, and the results were analyzed by using Biacore T200 Evaluation Software. Among them, the ATE antibody was used as a positive control (the amino acid sequence of the ATE antibody is identical to that of Atezolizumab, which is expressed by HEK293F cells).

Preparation of PD-L1-His antigen: the PD-L1 recombinant protein was constructed by adding 8×HIS tag at the C terminus of the extracellular domain (ECD) protein of human PD-L1, and the ECD region of human PD-L1 was genetically synthesized and subcloned into a mammalian expression vector (such as pcDNA3.1(+) expression vector). After transient transfection of HEK293F cells, a PD-L1-His antigen purified by nickel-based immobilized metal affinity chromatography (GE Healthcare) was obtained. The amino acid sequence of the extracellular domain (ECD) of human PD-L1 was: 
MRIFAVFIFMTYWHLLNAFTVTVPKDLYVVEYGSNMTIECKFPVEKQLDLAALIVYW
EMEDKNIIQFVHGEEDLKVQHSSYRQRARLLKDQLSLGNAALQITDVKLQDAGVYR
CMISYGGADYKRITVKVNAPYNKINQRILVVDPVTSEHELTCQAEGYPKAEVIWTSSD
HQVLSGKTTTTNSKREEKLFNVTSTLRINTTTNEIFYCTFRRLDPEENHTAELVIPELPL
AHPPNER (SEQ ID NO: 98).
1) As shown in Table 14, the affinity of the antibodies disclosed herein (L1-R2-4, L1-R2-18, L1-R2-78, and L1-R2-89) for the PD-L1 antigen was substantially similar as compared to the ATE antibody.

**Table 14. Dissociation constants of antibodies**

| Antibody No. | KD (M) |
|---|---|
| L1-R2-4 | 1.67E-09 |
| L1-R2-6 | 1.03E-08 |
| L1-R2-18 | 3.92E-09 |
| L1-R2-78 | 8.99E-09 |
| L1-R2-85 | 1.15E-08 |
| L1-R2-89 | 4.08E-09 |
| ATE | 1.12E-09 |

2) As shown in Table 15, the affinity of the antibodies disclosed herein (L1-R2-4-36, L1-R2-4-46, L1-R2-4-47, L1-R2-4-55, and L1-R2-4-71) for the PD-L1 antigen was higher as compared to the ATE antibody.

**Table 15. Dissociation constants of antibodies**

| Antibody No. | KD (M) |
|---|---|
| L1-R2-4-36 | 3.41E-10 |
| L1-R2-4-46 | 2.82E-10 |
| L1-R2-4-47 | 2.90E-10 |
| L1-R2-4-55 | 3.71E-10 |
| L1-R2-4-71 | 2.82E-10 |
| ATE | 1.84E-09 |

### Example 3. Detection of Anti-PD-L 1 Antibody Activity

Anti-PD-1/PD-L1 antibodies can relieve the inhibitory effect on a downstream NFAT signaling pathway by blocking the binding of PD-1 to PD-L1. The mechanisms of action (MOA) detection system (PD-1/PD-L1 Blockade Bioassay, Propagation Model, Catalog J1252) available from Promega, Inc. was used. According to the method provided in the product manual, the activation of NFAT signal was reflected by detecting the expression of luciferase reporter genes, thereby detecting the inhibitory effects of the antibodies on the binding of PD-1/PD-L1. IgG-Isotype, a negative control antibody, was purchased from Sino Biological Inc. (Cat. No. HG1K).

CHO-PD-L1 cells (from the MOA detection system described above) were plated the day before the activity assay, and were passaged 1-2 days before plating CHO-PD-L1 cells. The culture supernatant was discarded, and the cells were washed once with sterile PBS. An appropriate amount of Trypsin (Gibco) was added for digesting in an incubator at 37 °C with 5% CO₂ for 3-5 min. Fresh culture medium was added to terminate the digestion, and the cells were transferred to a 50 mL centrifuge tube and counted. A desired volume of cells was taken and centrifuged at 900 rpm for 5 min. The cells were resuspended to 4 × 10⁵ cells/mL with DMEM-F12 medium (Gibco). The cells were added to a 96-well white cell culture plate (Corning) at 100 µL/well. The cells were incubated in an incubator at 37 °C with 5% CO₂ overnight.

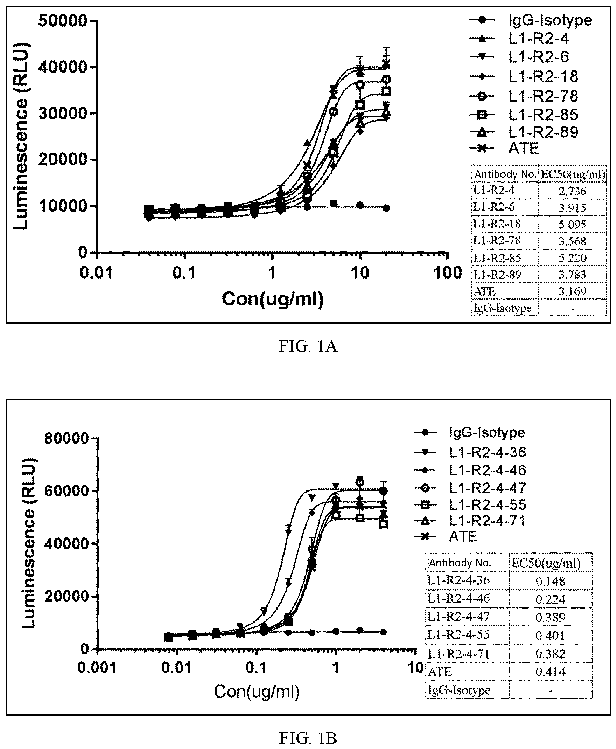

Jurkat-PD1 cells (from the MOA detection system described above) were treated on the day of detection: after counting, a desired volume of cells was taken and centrifuged at 900 rpm for 5 min. The cells were resuspended to 1.25 × 10⁶ cells/mL with assay buffer (1640 medium (Gibco) + 1% FBS) for later use. 95 µL/well of the culture supernatant in the CHO-PD-L1 cell culture plate was discarded, 40 µL/well of the test samples (the anti-PD-L1 antibodies, ATE antibody or negative control HG1K antibody described above with the highest concentration of 20 µg/mL were 2-fold diluted in assay buffer for 9 gradients) were added, and 40 µL of Jurkat-PD1 cells were then added, mixed by gentle shaking and incubated in an incubator at 37 °C with 5% CO₂ for 6 h. Detection: ONE-Glo^{™} Luciferase Assay System (purchased from Promega, E6120) was thawed in advance. After 6 h, ONE-Glo^{™} reagent was added at 50 µL/well. The mixture was let stand at room temperature for 5-10 min, and then the result was read.
1) As shown in FIG. 1A, the anti-PD-L1 antibodies of this example (L1-R2-4, L1-R2-6, L1-R2-18, L1-R2-78, L1-R2-85, and L1-R2-89) all could effectively block the PD-1/PD-L1 interaction; wherein the L1-R2-4 antibody had stronger blocking activity.
2) As shown in FIG. 1B, the antibodies of the present invention (L1-R2-4-36, L1-R2-4-46, L1-R2-4-47, L1-R2-4-55, and L1-R2-4-71) all could effectively block the PD-1/PD-L1 interaction; compared with the control antibody ATE, the antibodies (L1-R2-4-36 and L1-R2-4-46) had stronger blocking activity.

### Example 4. Detection of Binding Force of Anti-PD-L1 Antibodies

The binding of anti-PD-L1 antibodies of the present invention to CHO cells overexpressing human PD-L1 was detected by flow cytometry. The test steps were as follows: CHO-PD-L1 cells (from the MOA detection system described above) were mixed with antibodies with different dilution concentrations (the highest working concentration of the antibodies was 20 nM, and the dilution ratio was 1:2 for 11 gradients) and incubated on ice for 1 h; the cells were washed twice with PBS, then a PE-labeled anti-human Fc antibody (Jackson ImmunoResearch) fluorescent secondary antibody was added, and incubated on ice away from light for 30 min; after washing twice with PBS, the cells were resuspended with PBS. The binding of the antibodies to the cells was detected by FACS.

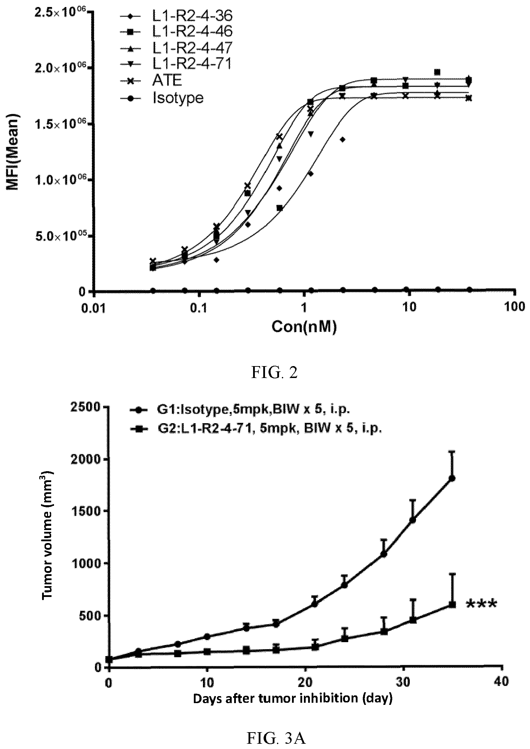

As shown in FIG. 2, the anti-PD-L1 antibodies of the present invention (L1-R2-4-36, L1-R2-4-46, L1-R2-4-47, L1-R2-4-55, and L1-R2-4-71) all could bind to human PD-L1 overexpressed on CHO cells.

### Example 5. Anti-Tumor Effect

Test mice: female C57BL/6 mice (aged 5-8 weeks) were used (GemPharmatech Co., Ltd.). Test cells: mouse colon cancer cells MC38 in logarithmic growth phase were collected, removed the culture medium, washed twice with PBS, and then inoculated into the right abdominal region in an amount of 1 × 10⁶/100 µL/mouse. Grouping and administration: when the average tumor volume was 50-100 mm³, the tumor-bearing mice were randomized into groups; enrollment criteria: the tumor volume CV value was less than 30%; the day of inoculation was defined as D0, and on the day of grouping, dosing was started according to the experimental protocol design. The administration dose and administration mode are shown in Table 16. After cell inoculation, the effect of tumors on the normal behavior of the animals was routinely monitored weekly. Specifically, routine monitoring included activity, food and water intake, weight gain or loss, eyes, hair and other abnormal conditions of the test animals. Clinical symptoms observed during the test were recorded in the raw data. After the administration was started, the body weight was measured twice a week. The tumor volume was measured twice a week. The tumor volume was calculated as follows: tumor volume (mm³) = 0.5 × long diameter of tumor × short diameter of tumor². The monitoring ended 35 days after inoculation. On day 35 after inoculation, the relative tumor growth inhibition (TGI%) was calculated by the following formula: TGI% = (1 - average relative tumor volume in treatment group/average relative tumor volume in vehicle group) × 100%.

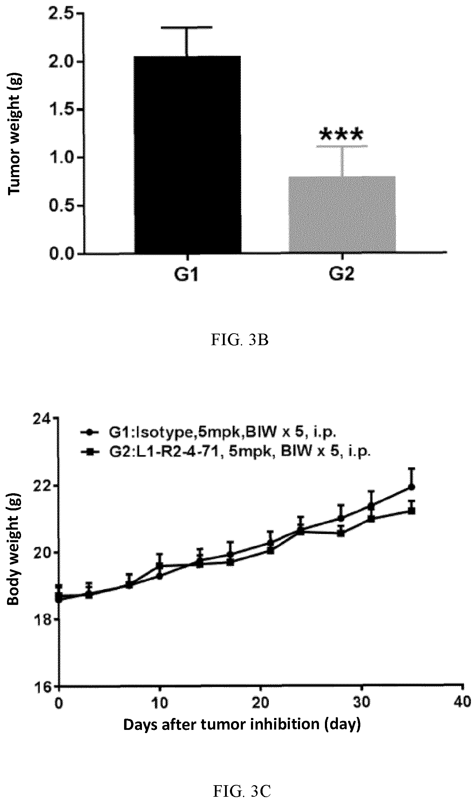

The results of tumor growth inhibition are shown in FIGs. 3A and 3B and Table 17. On day 35 after inoculation, compared with Isotype IgG1 group, the tumor growth inhibition of the L1-R2-4-71 antibody was about 70.89% at a single dose administration of 5 mg/kg, and the tumor weight was reduced by about 61.81%; the antibody of the present invention showed a significant anti-tumor effect one week after administration, and the tumor in 2 mice in the antibody group of the present invention (group G2) completely regressed at day 35. As shown in FIG. 3C, there was no significant difference in the body weight of the mice.

**Table 16. Experiment design**

| Group | Administration group | Dose (mg/kg) | Route | Frequency of administration/week |
|---|---|---|---|---|
| G1 | Isotype IgG1 | 5 | Intraperitoneal | BIW×5 |
| G2 | L1-R2-4-71 | 5 | Intraperitoneal | BIW×5 |

**Table 17. Tumor growth inhibition on day 35**

| Group | Administration group | Tumor volume (mm³) | Tumor growth inhibition (TGI%) |
|---|---|---|---|
| G1 | Isotype IgG1 | 1803.59 | N/A |
| G2 | L1-R2-4-71 | 592.40 | 70.89% |

### Example 6. Anti-PD-L1 Antibodies Displayed by Yeast in the Form of scFv

Nucleic acid sequences encoding scFv fragments (including a heavy chain variable regi on, a light chain variable region, and linker of the heavy chain variable region and th e light chain variable region (G₄S)₃) were synthesized, inserted into pYD1 vector (Add gene) through restriction site endonuclease, and transferred into host saccharomyces cer evisiae (e.g., Invitrogen, EBY100) by an electroporation apparatus to obtain positive ye ast clones. The positive yeast clones obtained above were picked up and subjected to monoclonal culture. The heavy chain variable region and the light chain variable regio n of the scFV expressed on the surface of the ATE positive yeast clone were consiste nt with those of Atezolizumab, and the scFVs expressed on the surfaces of other posi tive yeast clones are shown in Table 18. The amino acid sequence of the linker was GGGGSGGGGSGGGGS (SEQ ID NO: 106), and the nucleic acid sequence was GGT GGAGGCGGTTCAGGCGGAGGTGGCTCTGGCGGTGGCGGATCG (SEQ ID NO: 10 7). Taking CP11-46 as an example, the nucleic acid sequences of VH and VL thereof are shown in Table 13. Taking CP11-71 as an example, the nucleic acid sequences of VH and VL thereof are shown in Table 13.

**Table 18. scFv displayed by positive yeast clones (VH at N terminus of scFV)**

| scFV | VH sequence No. | VL sequence No. |
|---|---|---|
| CP11-36 | 37 | 68 |
| CP11-46 | 38 | 68 |
| CP11-47 | 39 | 68 |
| CP11-55 | 40 | 68 |
| CP11-71 | 41 | 68 |
| CP14-16 | 42 | 68 |
| CP14-80 | 43 | 68 |
| CP21-8 | 44 | 68 |
| CP21-47 | 45 | 68 |
| CP21-59 | 46 | 68 |
| CP22-34 | 47 | 68 |
| CP22-40 | 48 | 68 |
| CP22-44 | 49 | 68 |
| CP22-45 | 50 | 68 |

### Example 7. Binding Force of Anti-PD-L1 scFv Displayed by the Yeast to Antigen

The binding force of the anti-PD-L1 scFv displayed by the yeast to the antigen protein PD-L1-His-Biotin was detected by flow cytometry. The yeasts described above were incubated with 1 nM of PD-L1-His-Biotin antigen protein at room temperature for 1 h, washed 3 times with PBS at pH 7.4, then incubated with Streptavidin-PE fluorescent secondary antibody at room temperature for 30 min, and washed 3 times with PBS at pH 7.4. The fresh PBS was added, and loaded on the machine for assay.

The preparation of PD-L1-His-Biotin: The purified PD-L1-His antigen protein was added with 26.6 µL/10 nM of biotin (Sigma-Aldrich, B4501-1G) prepared in DMSO per 1 mg of protein, incubated at room temperature for 2 h, and then dialyzed with PBS at pH 7.4.

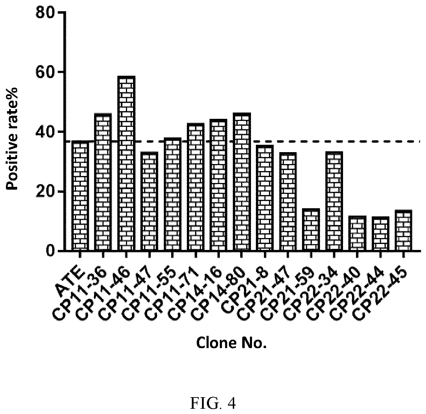

As shown in FIG. 4, the anti-PD-L1 scFv of the present invention (CP11-36, CP 11-46, CP11-71, CP14-16 and CP14-80) had significant better binding force to PD-L1-His-Biotin antigen protein than that of the positive control ATE scFv; compared with the positive control ATE scFV, the anti-PD-L1 scFV of the present invention (CP-11-47, CP-11-55, CP21-8, CP21-47 and CP22-34) had basically the same binding force to PD-L1-His-Biotin antigen protein.

## Claims

1. An antibody or an antigen-binding fragment, wherein the antibody or the antigen-binding fragment specifically binds to PD-L1, and comprises:
(a) an HCDR1 comprising an amino acid sequence set forth in X₁₅X₁₆X₁₇X₁₈X₁₉DSWIH, wherein X₁₅ is G, S, or N, X₁₆ is F, W, L, or S, X₁₇ is T, S, A, R, or H, X₁₈ is F, L, T, or P, and X₁₉ is S, K, R, or A; and/or
(b) an HCDR2 comprising GWISPYGGSTYYADX₂₀X₂₁X₂₂X₂₃, wherein X₂₀ is S, D, H, G, P, or Y, X₂₁ is V, F, L, M, or Y, X₂₂ is K, R, G, S, V, or H, and X₂₃ is G, H, D, Q, S, or A; and/or
(c) an HCDR3 comprising RHWPGGX₂₄X₂₅X₂₆, wherein X₂₄ is F or L, X₂₅ is D or L, and X₂₆ is Y or P; and/or
(d) an LCDR1 comprising X₁ASQX₂IX₃X₄X₅LX₆, wherein X₁ is L, Q, or R, X₂ is D, T, or G, X₃ is G or S, X₄ is K, T, or S, X₅ is H, W, F, or Y, and X₆ is N or A; and/or
(e) an LCDR2 comprising X₇ASX₈LX₉X₁₀, wherein X₇ is A or G, X₈ is T, N, S, or R, X₉ is Q or K, and X₁₀ is S or T; and/or
(f) an LCDR3 comprising QQX₁₁X₁₂X₁₃TPX₁₄T, wherein X₁₁ is Y or S, X₁₂ is Y or F, X₁₃ is S or T, and X₁₄ is R or Y

2. The antibody or the antigen-binding fragment according to claim 1, wherein the HCDR1 comprises an amino acid sequence set forth in any one of SEQ ID NOs: 1-6, the HCDR2 comprises an amino acid sequence set forth in any one of SEQ ID NOs: 7-16, and the HCDR3 comprises an amino acid sequence set forth in SEQ ID NO: 17 or 18.

3. The antibody or the antigen-binding fragment according to claim 1 or 2, wherein the LCDR1 comprises an amino acid sequence set forth in any one of SEQ ID NOs: 19-23, the LCDR2 comprises an amino acid sequence set forth in any one of SEQ ID NOs: 24-27, and the LCDR3 comprises an amino acid sequence set forth in any one of SEQ ID NOs: 28-31.

4. The antibody or the antigen-binding fragment according to any one of claims 1-3, wherein the antibody or the antigen-binding fragment at least comprises the HCDR1 set forth in SEQ ID NO: 1, the HCDR2 set forth in SEQ ID NO: 8, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28; and/or
the antibody or the antigen-binding fragment at least comprises the HCDR1 set forth in SEQ ID NO: 1, the HCDR2 set forth in SEQ ID NO: 11, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28; and/or
the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28; and/or
the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 20, the LCDR2 set forth in SEQ ID NO: 25, and the LCDR3 set forth in SEQ ID NO: 29; and/or
the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 21, the LCDR2 set forth in SEQ ID NO: 26, and the LCDR3 set forth in SEQ ID NO: 30; and/or
the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 22, the LCDR2 set forth in SEQ ID NO: 27, and the LCDR3 set forth in SEQ ID NO: 31; and/or
the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 23, the LCDR2 set forth in SEQ ID NO: 26, and the LCDR3 set forth in SEQ ID NO: 30; and/or
the antibody or the antigen-binding fragment at least comprises the HCDR1 set forth in SEQ ID NO: 1, the HCDR2 set forth in SEQ ID NO: 16, the HCDR3 set forth in SEQ ID NO: 17, the LCDR1 set forth in SEQ ID NO: 19, the LCDR2 set forth in SEQ ID NO: 26, and the LCDR3 set forth in SEQ ID NO: 31; and/or
the antibody or the antigen-binding fragment at least comprises the HCDR1 set forth in SEQ ID NO: 1, the HCDR2 set forth in SEQ ID NO: 8, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28; and/or
the antibody or the antigen-binding fragment at least comprises the HCDR1 set forth in SEQ ID NO: 1, the HCDR2 set forth in SEQ ID NO: 9, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28; and/or
the antibody or the antigen-binding fragment at least comprises the HCDR1 set forth in SEQ ID NO: 1, the HCDR2 set forth in SEQ ID NO: 10, the HCDR3 set forth in SEQ ID NO: 17, the LCDR1 set forth in SEQ ID NO: 19, the LCDR2 set forth in SEQ ID NO: 24, and the LCDR3 set forth in SEQ ID NO: 28.

5. The antibody or the antigen-binding fragment according to any one of claims 1-4, wherein a heavy chain of the antibody or the antigen-binding fragment further comprises FR1, FR2, FR3, and FR4, wherein the heavy chain FR1 comprises a sequence set forth in SEQ ID NO: 32, a sequence having at least 90% identity to the sequence set forth in SEQ ID NO: 32, or an amino acid sequence having one or more conservative amino acid substitutions as compared to the sequence set forth in SEQ ID NO: 32; and/or
the heavy chain FR2 of the antibody or the antigen-binding fragment comprises a sequence set forth in SEQ ID NO: 33, a sequence having at least 90% identity to the sequence set forth in SEQ ID NO: 33, or an amino acid sequence having one or more conservative amino acid substitutions as compared to the sequence set forth in SEQ ID NO: 33; and/or
the heavy chain FR3 of the antibody or the antigen-binding fragment comprises a sequence set forth in SEQ ID NO: 34, a sequence having at least 90% identity to the sequence set forth in SEQ ID NO: 34, or an amino acid sequence having one or more conservative amino acid substitutions as compared to the sequence set forth in SEQ ID NO: 34; and/or
the heavy chain FR4 of the antibody or the antigen-binding fragment comprises a sequence set forth in SEQ ID NO: 35, a sequence having at least 90% identity to the sequence set forth in SEQ ID NO: 35, or an amino acid sequence having one or more conservative amino acid substitutions as compared to the sequence set forth in SEQ ID NO: 35.

6. The antibody or the antigen-binding fragment according to claim 5, wherein the heavy chain FR1 of the antibody or the antigen-binding fragment comprises a sequence set forth in SEQ ID NO: 32, the heavy chain FR2 of the antibody or the antigen-binding fragment comprises a sequence set forth in SEQ ID NO: 33, the heavy chain FR3 of the antibody or the antigen-binding fragment comprises a sequence set forth in SEQ ID NO: 34, and the heavy chain FR4 of the antibody or the antigen-binding fragment comprises a sequence set forth in SEQ ID NO: 35.

7. The antibody or the antigen-binding fragment according to any one of claims 1-6, wherein a light chain of the antibody or the antigen-binding fragment further comprises FR1, FR2, FR3, and FR4, wherein the light chain FR1 comprises a sequence set forth in any one of SEQ ID NOs: 51-56, a sequence having at least 90% identity to the sequence set forth in any one of SEQ ID NOs: 51-56, or an amino acid sequence having one or more conservative amino acid substitutions as compared to the sequence set forth in SEQ ID NOs: 51-56; and/or
the light chain FR2 of the antibody or the antigen-binding fragment comprises a sequence set forth in any one of SEQ ID NOs: 57-60, a sequence having at least 90% identity to the sequence set forth in any one of SEQ ID NOs: 57-60, or an amino acid sequence having one or more conservative amino acid substitutions as compared to the sequence set forth in any one of SEQ ID NOs: 57-60; and/or
the light chain FR3 of the antibody or the antigen-binding fragment comprises a sequence set forth in any one of SEQ ID NOs: 61-64, a sequence having at least 90% identity to the sequence set forth in any one of SEQ ID NOs: 61-64, or an amino acid sequence having one or more conservative amino acid substitutions as compared to the sequence set forth in any one of SEQ ID NOs: 61-64; and/or
the light chain FR4 of the antibody or the antigen-binding fragment comprises a sequence set forth in any one of SEQ ID NOs: 65-67, a sequence having at least 90% identity to the sequence set forth in any one of SEQ ID NOs: 65-67, or an amino acid sequence having one or more conservative amino acid substitutions as compared to the sequence set forth in any one of SEQ ID NOs: 65-67.

8. The antibody or the antigen-binding fragment according to claim 7, wherein the light chain FR1 of the antibody or the antigen-binding fragment comprises a sequence set forth in SEQ ID NO: 51, the light chain FR2 of the antibody or the antigen-binding fragment comprises a sequence set forth in SEQ ID NO: 57, the light chain FR3 of the antibody or the antigen-binding fragment comprises a sequence set forth in SEQ ID NO: 61, and the light chain FR4 of the antibody or the antigen-binding fragment comprises a sequence set forth in SEQ ID NO: 65; or
the light chain FR1 of the antibody or the antigen-binding fragment comprises a sequence set forth in SEQ ID NO: 52, the light chain FR2 of the antibody or the antigen-binding fragment comprises a sequence set forth in SEQ ID NO: 58, the light chain FR3 of the antibody or the antigen-binding fragment comprises a sequence set forth in SEQ ID NO: 62, and the light chain FR4 of the antibody or the antigen-binding fragment comprises a sequence set forth in SEQ ID NO: 66; or
the light chain FR1 of the antibody or the antigen-binding fragment comprises a sequence set forth in SEQ ID NO: 53, the light chain FR2 of the antibody or the antigen-binding fragment comprises a sequence set forth in SEQ ID NO: 59, the light chain FR3 of the antibody or the antigen-binding fragment comprises a sequence set forth in SEQ ID NO: 63, and the light chain FR4 of the antibody or the antigen-binding fragment comprises a sequence set forth in SEQ ID NO: 67; or
the light chain FR1 of the antibody or the antigen-binding fragment comprises a sequence set forth in SEQ ID NO: 54, the light chain FR2 of the antibody or the antigen-binding fragment comprises a sequence set forth in SEQ ID NO: 60, the light chain FR3 of the antibody or the antigen-binding fragment comprises a sequence set forth in SEQ ID NO: 64, and the light chain FR4 of the antibody or the antigen-binding fragment comprises a sequence set forth in SEQ ID NO: 67; or
the light chain FR1 of the antibody or the antigen-binding fragment comprises a sequence set forth in SEQ ID NO: 55, the light chain FR2 of the antibody or the antigen-binding fragment comprises a sequence set forth in SEQ ID NO: 59, the light chain FR3 of the antibody or the antigen-binding fragment comprises a sequence set forth in SEQ ID NO: 63, and the light chain FR4 of the antibody or the antigen-binding fragment comprises a sequence set forth in SEQ ID NO: 67; or
the light chain FR1 of the antibody or the antigen-binding fragment comprises a sequence set forth in SEQ ID NO: 56, the light chain FR2 of the antibody or the antigen-binding fragment comprises a sequence set forth in SEQ ID NO: 57, the light chain FR3 of the antibody or the antigen-binding fragment comprises a sequence set forth in SEQ ID NO: 61, and the light chain FR4 of the antibody or the antigen-binding fragment comprises a sequence set forth in SEQ ID NO: 67.

9. The antibody or the antigen-binding fragment according to any one of claims 1-8, wherein a heavy chain variable region of the antibody or the antigen-binding fragment comprises a sequence set forth in any one of SEQ ID NOs: 36-50, a sequence having at least 80% identity to the sequence set forth in any one of SEQ ID NOs: 36-50, or an amino acid sequence having one or more conservative amino acid substitutions as compared to the sequence set forth in any one of SEQ ID NOs: 36-50; and/or
a light chain variable region of the antibody or the antigen-binding fragment comprises a sequence set forth in any one of SEQ ID NOs: 68-73, a sequence having at least 80% identity to the sequence set forth in any one of SEQ ID NOs: 68-73, or an amino acid sequence having one or more conservative amino acid substitutions as compared to the sequence set forth in any one of SEQ ID NOs: 68-73.

10. The antibody or the antigen-binding fragment according to any one of claims 1-9, wherein the heavy chain variable region of the antibody or the antigen-binding fragment comprises a sequence set forth in any one of SEQ ID NOs: 36-50, and the light chain variable region of the antibody or the antigen-binding fragment comprises a sequence set forth in any one of SEQ ID NOs: 68-73.

11. The antibody or the antigen-binding fragment according to any one of claims 1-10, wherein the heavy chain variable region of the antibody or the antigen-binding fragment comprises a sequence set forth in any one of SEQ ID NOs: 36-50, and the light chain variable region of the antibody or the antigen-binding fragment comprises a sequence set forth in any one of SEQ ID NOs: 68-73; and/or
the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 68; and/or
the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 69; and/or
the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 70; and/or
the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 71; and/or
the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 72; and/or
the heavy chain variable region of the antibody or the antigen-binding fragment comprises a sequence set forth in SEQ ID NO: 36, and the light chain variable region of the antibody or the antigen-binding fragment comprises a sequence set forth in SEQ ID NO: 73; and/or
the heavy chain variable region of the antibody or the antigen-binding fragment comprises a sequence set forth in SEQ ID NO: 37, and the light chain variable region of the antibody or the antigen-binding fragment comprises a sequence set forth in SEQ ID NO: 68; and/or
the heavy chain variable region of the antibody or the antigen-binding fragment comprises a sequence set forth in SEQ ID NO: 38, and the light chain variable region of the antibody or the antigen-binding fragment comprises a sequence set forth in SEQ ID NO: 68; and/or
the heavy chain variable region of the antibody or the antigen-binding fragment comprises a sequence set forth in SEQ ID NO: 39, and the light chain variable region of the antibody or the antigen-binding fragment comprises a sequence set forth in SEQ ID NO: 68; and/or
the heavy chain variable region of the antibody or the antigen-binding fragment comprises a sequence set forth in SEQ ID NO: 40, and the light chain variable region of the antibody or the antigen-binding fragment comprises a sequence set forth in SEQ ID NO: 68; and/or
the heavy chain variable region of the antibody or the antigen-binding fragment comprises a sequence set forth in SEQ ID NO: 41, and the light chain variable region of the antibody or the antigen-binding fragment comprises a sequence set forth in SEQ ID NO: 68.

12. The antibody or the antigen-binding fragment according to any one of claims 1-11, wherein a heavy chain constant region of the antibody or the antigen-binding fragment comprises a sequence having an amino acid sequence set forth in SEQ ID NO: 74 or 75, a sequence having at least 80% identity to the sequence set forth in SEQ ID NO: 74 or 75, or an amino acid sequence having one or more conservative amino acid substitutions as compared to the sequence set forth in SEQ ID NO: 74 or 75; and/or
a light chain constant region of the antibody or the antigen-binding fragment comprises a sequence having an amino acid sequence set forth in SEQ ID NO: 76, a sequence having at least 80% identity to the sequence set forth in SEQ ID NO: 76, or an amino acid sequence having one or more conservative amino acid substitutions as compared to the sequence set forth in SEQ ID NO: 76.

13. The antibody or the antigen-binding fragment according to any one of claims 1-12, wherein the heavy chain constant region of the antibody or the antigen-binding fragment comprises a sequence having an amino acid sequence set forth in SEQ ID NO: 74, and the light chain constant region of the antibody or the antigen-binding fragment comprises a sequence having an amino acid sequence set forth in SEQ ID NO: 76; or
the heavy chain constant region of the antibody or the antigen-binding fragment comprises a sequence having an amino acid sequence set forth in SEQ ID NO: 75, and the light chain constant region of the antibody or the antigen-binding fragment comprises a sequence having an amino acid sequence set forth in SEQ ID NO: 76.

14. An antibody or an antigen-binding fragment, wherein a heavy chain of the antibody comprises a sequence having an amino acid sequence set forth in any one of SEQ ID NOs: 77-91, a sequence having at least 80% identity to the sequence set forth in any one of SEQ ID NOs: 77-91, or an amino acid sequence having one or more conservative amino acid substitutions as compared to the sequence set forth in any one of SEQ ID NOs: 77-91; and/or
a light chain of the antibody comprises a sequence having an amino acid sequence set forth in any one of SEQ ID NOs: 92-97, a sequence having at least 80% identity to the sequence set forth in any one of SEQ ID NOs: 92-97, or an amino acid sequence having one or more conservative amino acid substitutions as compared to the sequence set forth in SEQ ID NOs: 92-97.

15. The antibody or the antigen-binding fragment according to claim 14, wherein the heavy chain of the antibody comprises a sequence having an amino acid sequence set forth in SEQ ID NO: 77, and the light chain of the antibody comprises a sequence having an amino acid sequence set forth in SEQ ID NO: 92; or
the heavy chain of the antibody comprises a sequence having an amino acid sequence set forth in SEQ ID NO: 77, and the light chain of the antibody comprises a sequence having an amino acid sequence set forth in SEQ ID NO: 93; or
the heavy chain of the antibody comprises a sequence having an amino acid sequence set forth in SEQ ID NO: 77, and the light chain of the antibody comprises a sequence having an amino acid sequence set forth in SEQ ID NO: 94; or
the heavy chain of the antibody comprises a sequence having an amino acid sequence set forth in SEQ ID NO: 77, and the light chain of the antibody comprises a sequence having an amino acid sequence set forth in SEQ ID NO: 95; or
the heavy chain of the antibody comprises a sequence having an amino acid sequence set forth in SEQ ID NO: 77, and the light chain of the antibody comprises a sequence having an amino acid sequence set forth in SEQ ID NO: 96; or
the heavy chain of the antibody comprises a sequence having an amino acid sequence set forth in SEQ ID NO: 77, and the light chain of the antibody comprises a sequence having an amino acid sequence set forth in SEQ ID NO: 97; or
the heavy chain of the antibody comprises a sequence having an amino acid sequence set forth in SEQ ID NO: 78, and the light chain of the antibody comprises a sequence having an amino acid sequence set forth in SEQ ID NO: 92; or
the heavy chain of the antibody comprises a sequence having an amino acid sequence set forth in SEQ ID NO: 79, and the light chain of the antibody comprises a sequence having an amino acid sequence set forth in SEQ ID NO: 92; or
the heavy chain of the antibody comprises a sequence having an amino acid sequence set forth in SEQ ID NO: 80, and the light chain of the antibody comprises a sequence having an amino acid sequence set forth in SEQ ID NO: 92; or
the heavy chain of the antibody comprises a sequence having an amino acid sequence set forth in SEQ ID NO: 81, and the light chain of the antibody comprises a sequence having an amino acid sequence set forth in SEQ ID NO: 92; or
the heavy chain of the antibody comprises a sequence having an amino acid sequence set forth in SEQ ID NO: 82, and the light chain of the antibody comprises a sequence having an amino acid sequence set forth in SEQ ID NO: 92.

16. A pharmaceutical composition, comprising the antibody or the antigen-binding fragment according to any one of claims 1-15, and a pharmaceutically acceptable excipient.

17. A nucleic acid molecule, encoding the antibody or the antigen-binding fragment according to any one of claims 1-15.

18. A vector or a host cell, comprising the nucleic acid molecule according to claim 17.

19. A method for treating or ameliorating a T cell dysfunctional disorder, wherein the method comprises administering to a patient an effective dose of the antibody or the antigen-binding fragment according to any one of claims 1-15.

20. The method according to claim 19, wherein the T cell dysfunctional disorder includes, but is not limited to, an infection caused by bacteria, viruses, fungi or protozoa, as well as a cancer and a tumor; the cancer and the tumor are selected from the group consisting of: breast cancer, lung cancer, colon cancer, ovarian cancer, melanoma, bladder cancer, renal cancer, liver cancer, salivary gland cancer, gastric cancer, neuroglioma, thyroid cancer, thymus cancer, epithelial cancer, head cancer, neck cancer, and pancreatic cancer.

21. A diagnostic or prognostic kit, comprising the antibody or the antigen-binding fragment according to any one of claims 1-15.
